(19)

**Europäisches Patentamt**

**European Patent Office**

**Office européen des brevets**

(11)  **EP 3 024 942 B1**

(12)  **FASCICULE DE BREVET EUROPEEN**

(45) Date de publication et mention
de la délivrance du brevet:
**07.06.2017 Bulletin 2017/23**

(21) Numéro de dépôt: **14759003.8**

(22) Date de dépôt: **23.07.2014**

(51) Int Cl.:
*C12Q 1/04* (2006.01)      *G01N 33/68* (2006.01)

(86) Numéro de dépôt international:
**PCT/FR2014/051902**

(87) Numéro de publication internationale:
**WO 2015/011409 (29.01.2015 Gazette 2015/04)**

(54) **MÉTHODE DE DÉTECTION DE LA CONTAMINATION PAR UN MICROBE D'UN ÉCHANTILLON DE PRODUIT SANGUIN PAR ANALYSE DE SPECTRES DE MASSE**

VERFAHREN ZUR ERKENNUNG DER MIKROBIELLEN KONTAMINATION EINER BLUTPROBE DURCH MASSENSPEKTRALANALYSE

METHOD FOR DETECTING MICROBIAL CONTAMINATION OF A BLOOD SAMPLE PRODUCT BY MASS SPECTRUM ANALYSIS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.07.2013 FR 1357282**
**26.09.2013 FR 1359284**

(43) Date de publication de la demande:
**01.06.2016 Bulletin 2016/22**

(73) Titulaires:
• **Université d'Aix Marseille**
**13284 Marseille Cedex 07 (FR)**
• **Assistance Publique Hôpitaux de Marseille**
**13005 Marseille (FR)**
• **Etablissement Français du Sang**
**93218 La Plaine Saint Denis Cedex (FR)**
• **Fondation Méditerranée Infection**
**13005 Marseille (FR)**

(72) Inventeurs:
• **CAMOIN, Laurence**
**F-13112 La Destrousse (FR)**
• **CHABRIERE, Eric**
**F-13009 Marseille (FR)**

• **FLAUDROPS, Christophe**
**F-13009 Marseille (FR)**
• **RAOULT, Didier**
**F-13008Marseille (FR)**
• **CHIARONI, Jacques**
**F-13002 Marseille (FR)**
• **VIALATTE, Marie**
**F-13002 Marseille (FR)**
• **GALLIAN, Pierre**
**F-13009 Marseille (FR)**

(74) Mandataire: **Domange, Maxime**
**Cabinet Beau de Lomenie**
**232 Avenue du Prado**
**13008 Marseille (FR)**

(56) Documents cités:
**WO-A1-2012/016929      US-A1- 2012 107 864**

• **SAUER S ET AL: "Classification and identification of bacteria by mass spectrometry and computational analysis", PLOS ONE, vol. 3, no. 7, E2843, 30 juillet 2008 (2008-07-30), XP055114067, DOI: 10.1371/journal.pone.0002843**

EP 3 024 942 B1

**Description**

La présente invention concerne une méthode de détection rapide de contamination par un microbe d'un échantillon de produit sanguin à analyser, notamment par suivi de la pousse microbienne par analyse de spectre de spectrométrie de masse de type maldi-tof d'un extrait protéique dudit échantillon.

On entend ici par « produit sanguin », un produit provenant du sang contenant tout ou partie du sang, notamment un produit de transfusion, ou un produit destiné à complémenter le sang. Il s'agit le plus souvent d'un milieu liquide aqueux complexe contenant des protéines et/ou macromolécules lipidiques et/ou des composant insolubles du sang ou aptes à complémenter le sang chez certains patients tels que des cellules ou fragments de cellules et/ou des agrégats de dites protéines et/ou macromolécules. Des exemples sont donnés ci-après et notamment les concentrés de plaquettes qui sont des fragments de cellules dénommés thrombocytes ou des suspensions cellulaires aqueuses destinées à complémenter le sang d'un patient tel que des suspensions de cellules souches de la moelle osseuse encore appelées cellules hématopoïétiques, notamment pour le traitement des leucémies. Actuellement, l'identification et l'étude de la sensibilité des micro-organismes nécessitent plusieurs étapes qui reposent principalement sur la détection des caractéristiques phénotypiques du germe étudié. L'utilisation de colorations (coloration de Gram par exemple), la morphologie des colonies, l'examen au microscope, l'isolement en culture sur différents milieux, les tests biochimiques, qu'ils soient réalisés manuellement ou par des automates, sont les principes même de la classification et de l'identification des bactéries, des levures et des champignons. Ces approches, utilisées depuis de nombreuses années, permettent le rendu des résultats dans un laps de temps compris entre 24 h et 72 h, et nécessitent souvent que les micro-organismes présentent un métabolisme actif. Ce délai peut être allongé lorsque la culture est lente et/ou difficile ou lorsque les tests phénotypiques sont peu performants, ce qui est le cas avec les mycobactéries, les germes atypiques ou les anaérobies par exemple. Dans ces derniers cas, ce délai peut être de plusieurs jours à plusieurs semaines.

L'utilisation de la biologie moléculaire permet une identification bactérienne rapide et précise dans bien des cas par amplification d'ADN par PCR. Les avantages de la biologie moléculaire sont nombreux. Toutefois, les informations obtenues ne sont pas toujours suffisamment discriminantes pour permettre l'identification au niveau de l'espèce et l'étude d'autres gènes cibles est requise, ce qui augmente les délais de réponse. Outre les problèmes liés à son coût, cette méthodologie nécessite une grande expertise et doit être réservée aux identifications difficiles ou aux germes dont la culture est fastidieuse.

La spectrométrie de masse est une technique physique d'analyse permettant de détecter et d'identifier des molécules d'intérêt par mesure de leur masse. Son principe réside dans la séparation en phase gazeuse de molécules chargées (ions) en fonction de leur rapport masse/charge (m/z).Les applications de la spectrométrie de masse sont très vastes et concernent principalement l'identification de peptides ou de protéines, l'analyse de leur séquence en acides aminés ou encore la mise en évidence de modifications post-traductionnelles.

La spectrométrie de masse sous sa variante MALDI-TOF (matrix-assisted laser desorption ionization-time-of-flight) permet d'identifier et de classifier différents types bactériens en utilisant des bactéries intactes. En effet, parmi les divers composants identifiés dans un spectre de spectrométrie de masse MALDI-TOF, certains semblent spécifiques d'une espèce bactérienne précise. Ainsi, il a été possible d'identifier grand nombre d'espèces bactériennes dans des prélèvements cliniques en utilisant des banques de données fabriquées à partir de chacune de ces espèces. La spectrométrie de masse de type MALDI-TOF s'impose donc progressivement dans les laboratoires de microbiologie car ses atouts sont nombreux, rapidité et précision d'identification, et faible coût par échantillon.

Parmi les fabricants de spectromètre de masse, deux systèmes MALDI- TOF complets sont disponibles à la vente, commercialisés par les sociétés Bruker Daltonics (Allemagne) et Biomérieux (France). Chaque instrument est accompagné d'un logiciel de pilotage, d'une banque de données et d'un système expert permettant l'identification du micro-organisme reposant sur l'analyse du spectre généré par le spectromètre de masse.

Une application de cette technologie à l'identification des bactéries implique l'isolement et l'analyse de bactéries entières ou d'extraits protéiques de bactéries isolées, les dites bactéries étant isolées directement à partir des prélèvements, par exemple à partir de flacons d'hémocultures détectés comme positifs, ou à partir des urines. Le gain de temps est alors important pour la mise en route d'une antibiothérapie. Concernant les hémocultures, les résultats sont obtenus après des étapes intermédiaires de séparation des globules blancs des globules rouges. Les pourcentages d'identification correcte du genre varient de 72 % à 98 % selon les études. On retrouve les mêmes difficultés d'identification que celles que l'on rencontre lorsqu'on travaille à partir des colonies. Dans le cas des hémocultures polymicrobiennes, les spectres obtenus sont plus difficiles à interpréter et, dans le meilleur des cas, le germe prédominant est identifié. Pour les urines, les résultats requièrent d'avoir un inoculum bactérien supérieur à $10^5$ ufc/ml. Pour faciliter l'identification, les cultures obtenues en 24 h sont requises. Quant à l'identification des champignons filamenteux, elle requiert la présence de spores. Certaines études proposent la réalisation d'une étape supplémentaire d'extraction protéique avant l'analyse afin d'augmenter la qualité des spectres. Cette étape supplémentaire peut se justifier pour certaines espèces comme les streptocoques, ou en cas d'inoculum faible notamment pour les hémocultures ou les urines.

Actuellement, les types d'échantillons bactériens pouvant être analysés par un système MALDI-TOF sont classés en 2 catégories :

- les cultures bactériennes sur gélose et les cultures de levures, notamment de champignons filamenteux, qui se déposent directement sur la cible, et

- les hémocultures positives et les prélèvements urinaires, qui nécessitent une extraction de la bactérie préalable.

En pratique, la détection et l'identification d'agents microbiens sont donc réalisées en 2 étapes :

- une étape initiale d'isolement du microbe en milieu solide ou liquide

- une étape d'identification du microbe par spectrométrie Maldi-tof.

Actuellement, l'identification ne peut pas se faire directement sur un milieu liquide complexe sans étape d'isolement ou de séparation du microbe. De plus, il est nécessaire qu'un spectre de référence du microbe soit référencé dans une banque de données pour être identifié.

Un but de la présente invention concerne l'analyse d'un échantillon de produit sanguin, notamment un produit complexe issu du sang tel qu'un concentré de plaquettes comme décrit ci-après, pour une détection d'agents microbiens dans ce milieu complexe par spectrométrie Maldi-Tof sans étape d'isolement dans un délai de pas plus de 24h.

La transfusion du sang et celle de ses composants labiles isolés dénommés «produits sanguine labiles» (« PSL ») tels que les concentrés de globules rouges, concentrés de cellules souches hématopoïétiques , concentrés de plaquettes, ou du plasma frais éventuellement congelé ; et des médicaments préparés industriellement à partir de pools de centaines de plasma (albumine, immunoglobulines intraveineuses, facteurs de la coagulation) sont indispensables car, le plus souvent, il n'existe pas d'alternatives thérapeutiques.

Bien que le niveau de sécurité vis-à-vis des infections transmises par la transfusion soit élevé, le risque de transmission par transfusion d'une infection persiste. Il varie selon les produits sanguins labiles transfusés et il est accru chez le malade immunodéficitaire.

Les plaquettes sanguines sont les plus petits éléments figurés du sang. Elles sont produites dans la moelle osseuse comme les autres cellules sanguines. Les plaquettes jouent un rôle central dans les mécanismes de l'hémostase et plus particulièrement de l'hémostase primaire. Dans la circulation sanguine, les plaquettes sont indispensables pour que le sang coagule correctement en cas de brèche d'un vaisseau sanguin, afin de colmater celle-ci par la formation d'un caillot. Chez un individu sain, le nombre de plaquettes sanguines par litre de sang est compris entre 150 à 400 Giga ($10^9$). Toute anomalie quantitative (thrombopénie, taux de plaquette < 150 Giga/L) ou qualitative (thrombopathie) des plaquettes expose le patient à un risque hémorragique. La seule solution thérapeutique est de transfuser des plaquettes au patient sous forme de concentrés plaquettaires.

En France, c'est l'Etablissement Français du Sang (EFS) qui est responsable de la préparation des concentrés de plaquettes car l'administration de concentrés de plaquettes chez un patient est un acte de transfusion sanguine. Les concentrés de plaquettes sont obtenus par séparation primaire des thrombocytes du sang. Les plaquettes ont une durée de vie de 8 à 10 jours. On peut les concentrer à partir du sang total de plusieurs donneurs sous forme de mélange de concentrés de plaquettes («MCP ») ou les prélever chez un donneur unique par aphérèse pour obtenir un concentré dénommé concentré d'aphérèse («CPA»).

Des liquides de concentrés de plaquettes de ce type sous l'appellation ci-après de «concentré plaquettaire» ou «CP», sont connus et commercialisés avec des concentrations d'au moins $300.10^9$ plaquettes/l dans un soluté aqueux nutritif à base de sucre et de sel. Ils se conservent 5 jours à 20-24°C et sous agitation constante.

Les concentrés de plaquettes sont transfusés notamment dans les cas suivants :

- le nombre des plaquettes est très diminué (« thrombopénie ») parce que la moelle osseuse ne les fabrique plus ou qu'elles sont détruites dans la circulation ou dans la rate ;

- les plaquettes sont inefficaces parce que leur fonctionnement est altéré («thrombopathie») en raison d'une anomalie génétique ou à cause de certains traitements dont le plus connu est l'aspirine ;

- pour stopper une hémorragie déclarée ou de manière préventive, lorsque le taux de plaquettes est bas ou lorsque celles-ci n'assurent plus leur fonction coagulante, de sorte qu'un saignement grave peut survenir à n'importe quel moment ; et

- dans l'aplasie qui suit une chimiothérapie (il s'agit d'un traitement majeur).

Les concentrés de plaquettes («CP») sont propices à la prolifération d'un grand nombre de bactéries et constituent la catégorie de PSL la plus à risque au regard de transmission d'infections bactériennes provoquées par transfusion.

La présence de bactéries et leur prolifération au cours du stockage du PSL qui conduit non seulement à l'accroissement du nombre de bactéries mais également à la production de molécules toxiques (toxine et/ou endotoxines) représentant un risque vital pour le malade transfusé.

Bien qu'il ne soit pas possible de définir un seuil précis, une contamination supérieure à $10^5$ ufc/ml est considérée comme le seuil au-delà duquel l'ITCB (Infection Bactérienne Transmise par Transfusion) sera grave.

Trois méthodes de détection bactérienne dans les concentrés de plaquettes (CP) ont été proposés : BacTAlert® (Biomerieux, France), eBDS® (Pall Corporation, Etats-Unis) et Scan System® (Hemosystem, France). Ces trois techniques nécessitent pour être mises en oeuvre une période d'enrichissement d'une durée minimale de 24 heures, ce qui retarde d'autant la possibilité de délivrance des CP, dont la durée de conservation est limitée à cinq jours. BacTAlert® est une technique d'hémoculture automatisée avec lecture en temps réel. Le système eBDS® détecte la prolifération bactérienne par mesure de la consommation d'oxygène. De ce fait, il n'est pas adapté à la détection des bactéries anaérobies. Enfin, la technique ScanSystem® qui n'est plus disponible permet la détection directe en 70 minutes des bactéries par une méthode de cryométrie en phase solide.Cette technique nécessitait une technicité importante et un matériel complexe. Pour toutes ces techniques, le niveau de détection dépend du volume de la prise d'essai et il existe un risque important de défaut de représentativité de l'échantillon (pas de bactérie) qui rend l'enrichissement sans intérêt et conduit à un faux négatif. Aucune de ces techniques n'a permis de répondre de façon satisfaisante aux exigences d'une détection précoce, sensible et spécifique de dépistage des CP contaminés.

L'objectif de la présente invention est donc plus, particulièrement de détecter rapidement des microbes dans desdits produits sanguins et plus particulièrement dans des produits constitués d'un milieu liquide complexe contenant des composant insolubles, notamment des « CP », et ce sans étape d'isolement préalable du microbe.

Selon la présente invention on a découvert qu'il était possible d'obtenir des spectres exploitables par spectrométrie de masse de type maldi-tof d'échantillons de milieu complexe contenant des protéines ne provenant pas seulement d'une espèce bactérienne unique, sans isolement des microbes, exploitables pour détecter une différence de spectres entre échantillon non contaminé et échantillon contaminé comme explicité ci-après, pour autant que ledit échantillon subisse un traitement de lyse cellulaire au moins des microbes et de préférence un traitement une extraction de protéines après solubilisation des protéines qu'il contient.

On entend ici par «spectres exploitable», un spectre qui présente des pics vérifiant notamment les critères d'indice de validation ou de qualité dans les logiciels fournis avec le spectromètre mais aussi et surtout qui révèlent une combinaison de pics dont les positions et intensités soient spécifiques pour identifier ledit échantillon non contaminé.

L'obtention de tels spectres exploitable n'était pas possible avec les protocoles classiques d'analyse d'échantillon de milieu complexe sans isolement préalable du microbe utilisés dans l'état de l'art.

Plus précisément, la présente invention a pour objet une méthode de détection de la contamination par un microbe d'un échantillon de produit sanguin à analyser comprenant un milieu liquide contenant des protéines et/ou macromolécules et/ou des cellules et/ou fragments de cellules du sang ou destinés à complémenter le sang, dans lequel un dit microbe est susceptible de proliférer, ledit microbe étant de préférence une bactérie ou une levure, comprenant les étapes suivantes dans lesquelles :

a) on réalise un traitement de lyse d'au moins le ou les dits microbes pouvant être contenus dans ledit échantillon de dit produit sanguin à analyser après un temps t d'incubation et de préférence une étape d'extraction protéique du lysat,

b) on réalise un spectre par spectrométrie de masse de préférence du type MALDI-TOF dudit échantillon de dit produit sanguin ou d'un extrait protéique dudit échantillon de dit produit sanguin ainsi lysé à un temps t, et

c) on détermine si ledit échantillon à analyser est contaminé par comparaison du spectre obtenu avec au moins un spectre de spectrométrie de masse du type MALDI-TOF choisi parmi les spectres suivants :

c.1) au moins un spectre de référence choisi parmi :

- c.1.1 - au moins un spectre d'un dit échantillon standard ou respectivement d'un dit extrait protéique dudit échantillon standard, ledit échantillon standard ou respectivement ledit extrait de dit échantillon standard étant un échantillon de même nature que ledit échantillon à analyser, mais non contaminé, et

- c.1.2- des spectres de dits échantillons standards ou d'extraits protéiques d'échantillons standards de même nature contaminés chacun par respectivement un microbe connu différent, les dits échantillons standards ou dits extraits ayant subis le même traitement de lyse, et

c.2) au moins un spectre d'un dit échantillon d'origine ou dit extrait dudit échantillon d'origine ayant subi le même traitement de lyse avant incubation ou après un temps t' d'incubation différent de t.

On comprend qu'à l'étape a) on réalise un traitement dudit échantillon complet sans en extraire une partie contenant lesdits microbes et on réalise le traitement de lyse et/ou ladite extraction protéique sur ledit échantillon complet.

Plus particulièrement, la présente invention fournit une méthode de détection de la contamination par un microbe, de préférence une bactérie, d'un échantillon de dit produit sanguin à analyser par suivi de la croissance dudit microbe comprenant les étapes successives suivantes dans lesquelles :

a.1) on réalise une culture dudit microbe en ajoutant un milieu de culture dudit microbe dans dudit échantillon, pendant un temps t de préférence de 6 à 24 heures, de préférence encore pas plus de 16h, et

a.2) on réalise une lyse cellulaire des microbes et autres cellules dudit échantillon cellulaire, puis

a.3) on réalise une extraction des protéines à partir d'un lysat dudit échantillon après culture, de l'étape a.2), et

b) on réalise un spectre par spectrométrie de masse du type MALDI-TOF dudit extrait protéique obtenu à l'étape a.3), et

c) on détermine si ledit échantillon est contaminé par comparaison du spectre obtenu à l'étape b) avec au moins un spectre de spectrométrie de masse du type MALDI-TOF choisi parmi les spectres suivants :

c.1) au moins un spectre de référence choisi parmi :

- c.1.1) un spectre d'un extrait protéique de référence d'un échantillon standard de même nature mais non contaminé, ayant subi le même traitement de lyse et extraction des étapes a.2) et a.3), et

- c.1.2) des spectres de référence d'extraits protéiques d'échantillons standards de même nature contaminés par des microbes connus, ayant subis le même traitement au moins des étapes a.1) et a.2), de préférence ayant subis le même traitement au moins des a.1 à a.3), et

c.2) au moins un spectre d'un extrait protéique obtenu par extraction des protéines à partir d'un traitement de lyse et extraction dudit échantillon d'origine selon les étapes a.2) et a.3) avant ou après culture de l'étape a.1) pendant un temps t' inférieur à t.

On comprend que le cas échéant à l'étape a.1), on réalise ladite culture dans des conditions de culture aptes à permettre la croissance d'un dit microbe, de préférence en ajoutant un milieu de culture d'un dit microbe.

Selon la présente invention, on ne réalise pas une étape d'extraction dudit microbe entier à partir dudit échantillon, mais, à l'étape a.3), on extrait les protéines entières directement à partir d'un lysat de l'échantillon total. La lyse permet de lyser au moins les cellules des microbes pour ne retenir que les protéines.

Du fait de l'étape d'incubation ou de culture de l'étape a.1) préalable, les dites protéines sont en nombre suffisants pour permettre d'obtenir à l'étape c) des spectres exploitables.

A l'étape c.1), le spectre de référence dudit échantillon ne provient pas de l'échantillon d'origine à analyser. En effet, de façon originale et avantageuse, ledit spectre de référence est un spectre d'un extrait protéique de référence d'un échantillon standard de même nature mais non contaminé ou contaminé par un microbe déterminé connu et n'est donc pas un spectre de référence du dit microbe comme dans les méthodes usuelles car les pics spécifiques dudit microbe peuvent être en partie occultés par ceux des autres composants dudit échantillon.

A l'étape c.1), on détermine la présence de microbes contenu dans l'échantillon d'origine par une augmentation du nombre de pics dans le spectre de l'extrait de l'échantillon contaminé celui-ci comprenant des pics supplémentaires correspondant aux protéines ou composants spécifiques provenant du microbe en comparaison au spectre obtenu en l'absence de microbes dans l'échantillon de référence.

A l'étape c.2), on détermine la présence de microbe par la détection d'une croissance des microbes contenu dans l'échantillon d'origine entre l'extrait protéique obtenu à to avant culture ou incubation et celui obtenu à t1 supérieur à to après culture ou incubation et/ou entre les extraits protéiques obtenus après 2 temps de culture ou incubation t1 et t2 différents, cette croissance du microbe se traduisant par une augmentation de l'intensité des pics correspondant aux protéines ou composants provenant spécifiquement du microbe.

Inversement, comme mentionné aux étapes c.1) et c.2), la présence, voire la croissance, des microbes peut entrainer une dégradation par les dits microbes des composants spécifiques dudit échantillon d'origine tel que des cellules contenues dans l'échantillon d'origine autres que celles dudit microbe. Dans le cas où celui-ci est un échantillon biologique comme du sang, il peut s'agir notamment des érythrocytes ou leucocytes ; et pour les urines, il peut s'agir de leucocytes ou macromolécules contenus dans des urines. Toutefois, du fait que, selon l'invention, on analyse un lysat de préférence un extrait protéique dudit lysat, on n'observe en général pas de diminution significative du nombre des pics spécifique de l'échantillon autres que les pics spécifiques du microbe ou de baisse de l'intensité de ces pics spécifiques de l'échantillon non contaminés.

En tout état de cause, toute diminution relative éventuelle du nombre de protéines provenant des autres cellules ou autres composants de l'échantillon lui-même hors bactérie contaminante et/ou de l'intensité de leurs pics, sera prise en compte pour le calcul du différentiel des aires de spectres avant et après culture ou incubation.

La présente invention concerne avantageusement l'analyse d'extrait obtenu à partir d'un dit échantillon de produit sanguin à analyser constitué d'un milieu liquide complexe contenant des cellules ou fragments de cellules eucaryotes. On cite notamment des cellules du sang dans le cas de concentrés de globules rouges, et des thrombocytes dans le cas de concentrés de plaquettes, et des cellules de la moelle osseuse dans le cas de suspensions cellulaires destinées à complémenter le sang d'un patient tel que des suspensions de cellules hématopoïétiques, notamment pour le traitement des leucémies.

Plus particulièrement, ledit échantillon de produit sanguin est un échantillon humain ou animal contenant tout ou partie du sang choisi parmi le sang total, un concentré de globules rouges, un concentré de plaquettes ou du plasma frais éventuellement congelé. Il s'agit donc un échantillon biologique contenant différents types de protéines et différents types de cellules autres que celles de dits microbes, à savoir des cellules eucaryotes. Plus particulièrement encore, ledit échantillon est un liquide concentré de plaquettes de sang, de préférence contenant au moins $10.10^9$ plaquettes /l de soluté, de préférence encore au moins $100.10^9$ plaquettes /l.

Plus particulièrement encore, dans une première variante de réalisation, à l'étape c), on réalise l'étape c.1) en comparant le spectre de l'extrait protéique de lysat de concentré de plaquettes à analyser avec un spectre de référence qui est un spectre d'un extrait protéique de concentré de plaquettes standard non contaminé et on détecte une contamination si on retrouve dans le spectre de l'échantillon analysé au moins les pics spécifiques de plus grandes intensités du spectre de référence.

On a découvert selon la présente invention qu'il est possible de de déterminer un spectre de référence spécifiques des CP non contaminés si on analyse un extrait protéique de lysat cellulaire de CP, de préférence selon le protocole suivant.

Plus particulièrement encore, à l'étape a), ledit extrait protéique est obtenu en effectuant les étapes successives suivantes :

1) on élimine le liquide du dit échantillon à analyser, et on récupère un premier résidu solide, et

2) de préférence, on ajoute une solution de détergent au dit premier résidu solide, et

3) on élimine le liquide à nouveau et on récupère un deuxième résidu solide, et

4) on réalise une lyse des cellules présente dans ledit deuxième résidu solide, de préférence une lyse chimique, et

5) on solubilise les protéines présentes dans ledit deuxième résidu solide, de préférence avec une solution de solvant, et

6) on élimine les substances non solubilisées, pour récupérer une solution essentiellement constituée de protéines entières.

A l'étape 2), on supprime les interactions entre les dits microbes et les autres composants contenus dans ledit échantillon avec ladite solution de détergent ajoutée au dit premier résidu solide.

Plus particulièrement encore, pour traiter un échantillon de concentré de plaquettes, ledit extrait protéique est obtenu en effectuant les étapes successives suivantes :

1) on élimine le liquide du dit échantillon à analyser, en réalisant une première centrifugation et éliminant le surnageant de centrifugation, et en récupérant un premier résidu solide qui est le culot de première centrifugation, et

2) on ajoute une solution de détergent au dit premier résidu solide, de préférence une solution de saponine, et

3) on récupère un deuxième résidu solide par une deuxième centrifugation et en éliminant le surnageant et en récupérant un deuxième résidu solide qui est le culot de deuxième centrifugation, et

4) on réalise une lyse chimique des cellules présente dans ledit deuxième résidu solide, de préférence on réalise un traitement chimique avec une solution acide d'acide formique, et

5) on solubilise les protéines présentes dans ledit deuxième résidu solide de lysat cellulaire, de préférence avec une solution d'acide formique et acétonitrile, et

6) on élimine les substances non solubilisées, par une troisième centrifugation et en éliminant le culot et récupérant le surnageant de troisième centrifugation, pour récupérer une solution essentiellement constituée de protéines entières.

Concernant l'analyse comparative des spectres, à l'étape c), on considère qu'un pic est commun entre le spectre analysé et un profil ou spectre de référence, si le pic présente les critères de validation et de recalibration définis pour chaque modèle et connus de l'homme de l'art, et dont le paramétrage peut être modifié par l'opérateur selon les consignes explicitées dans les manuels d'utilisateur spécifiques des logiciels Bruker Daltonics® et tels que rappelé plus loin dans les exemples 1 et 4.

Lorsque l'on compare un nouveau spectre à un autre spectre, comme à l'étape c.1), ou à un profil de référence présent dans la base de données, comme à l'étape c.2) ci-dessus, on peut prendre en compte un autre score, appelé score d'identification décrit plus loin, calculé par le logiciel BIOTYPER®. Le logiciel BIOTYPER® de la société BRUCKER DALTONICS® peut identifier ainsi des spectres inconnus par comparaison avec des spectres de référence stockés dans une base de données.

Plus particulièrement, aux étapes c/, on ne prend en compte lesdits spectres de référence et spectres à analyser que si lesdits spectres présentent un score de qualité supérieure à 2, ledit score de qualité étant constitué de l'addition des deux notes ni et n2 ayant chacune une valeur de 0 à 6 avec :

- ni est la note attribuée en fonction du nombre de pics caractéristiques dudit spectre n, tel que spécifié plus précisément dans l'exemple 3 ci-après, et

- n2 est la note attribué en fonction de la valeur du rapport signal/bruit (Rmax) du pic de plus grande intensité dudit spectre, tel que spécifié plus précisément dans l'exemple 3 ci-après.

Les critères ci-dessus sont pris en compte à l'aide notamment du logiciel FLEX CONTROL®, BIOTYPER® RTC développés par le fabricant de spectromètre de masse Bruker Daltonik® GmbH, Leipzig, Germany de référence Microflex LT.

Plus particulièrement encore, les pics desdits spectres sont définis par une paire de coordonnées composées d'une abscisse constituée du rapport masse/charge (m/z) de 2 000 à 20000 Da, plus particulièrement de 3 000 à 16000 Da, la coordonnée en ordonnée étant une intensité relative ou en unité arbitraire, et on retient de préférence dans le spectre au moins les 70 pics de plus grandes intensités. On entend ici par "intensité relative", une intensité exprimée par le rapport de (S/N=intensité du pic/ intensité du bruit de fond).

Plus particulièrement encore, ledit spectre de référence spécifique de concentré de plaquettes sanguines non contaminé comprend au moins les pics caractéristiques de plus grande intensités dans le tableau 1 suivant dans lequel les différents pics caractéristiques sont caractérisés par leurs coordonnées en abscisse m/z (Da) et leurs coordonnées en ordonnée d'intensité relative exprimées en S/N (intensité du pic/intensité du bruit de fond), les valeurs des masses moléculaires m/z pouvant varier jusqu'à ±1 Da de la valeur moyenne mentionnée dans le tableau 1, et les valeurs d'intensité relative pouvant varier jusqu'à ±20% de la valeur moyenne d'intensité relative mentionnée:

Tableau 1 :

| Index | m/z | Intensité (S/N) | Index | m/z | Intensité (S/N) |
|-------|---------|-----------------|-------|---------|-----------------|
| 2 | 2039,33 | 0,82 | 45 | 5941,47 | 12,96 |
| 3 | 2053,09 | 0,96 | 46 | 6123,11 | 4,61 |
| 4 | 2172,47 | 2,81 | 47 | 6195,2 | 4,99 |
| 5 | 2188,26 | 2,08 | 48 | 6253,84 | 12,31 |
| 6 | 2206,14 | 1,49 | 49 | 6329,64 | 34,15 |

(suite)

| Index | m/z | Intensité (S/N) | Index | m/z | Intensité (S/N) |
|---|---|---|---|---|---|
| 7 | 2335,49 | 0,81 | 50 | 6399,07 | 4,69 |
| 8 | 2587,08 | 9,77 | 51 | 6437,89 | 19,1 |
| 9 | 2677,66 | 6,53 | 52 | 6636,41 | 10,92 |
| 10 | 2824,37 | 44,11 | 53 | 6667,28 | 30,17 |
| 11 | 3124,2 | 2,88 | 54 | 6725,81 | 41,44 |
| 12 | 3170,04 | 2,43 | 55 | 7193,2 | 13,26 |
| 13 | 3332,51 | 4,02 | 56 | 7307,12 | 1,57 |
| 14 | 3372,52 | 27,37 | 57 | 7355,33 | 2,03 |
| 15 | 3443,5 | 48,02 | 58 | 7437,27 | 19,85 |
| 16 | 3487,23 | 15,79 | 59 | 7567,73 | 42,56 |
| 17 | 3717,3 | 2,85 | 60 | 7642,27 | 28,22 |
| 18 | 3783,47 | 6,75 | 61 | 7768,13 | 745,58 |
| 19 | 3820,02 | 5,97 | 62 | 7839,16 | 152,91 |
| 20 | 3883,82 | 343,89 | 63 | 7924,93 | 114,32 |
| 21 | 3920,17 | 34,15 | 64 | 8022,69 | 21,31 |
| 22 | 3963,03 | 32,32 | 65 | 8144,94 | 177,92 |
| 23 | 4049,38 | 1,6 | 66 | 8211,76 | 12,77 |
| 24 | 4072,05 | 48,19 | 67 | 8584,21 | 37,68 |
| 25 | 4292,54 | 15,29 | 68 | 8652,46 | 28,04 |
| 26 | 4326,29 | 5,36 | 69 | 8719,83 | 16,44 |
| 27 | 4481,88 | 47,61 | 70 | 8963,52 | 106,86 |
| 28 | 4568,92 | 4,21 | 71 | 9039,83 | 6,86 |
| 29 | 4623,37 | 14,23 | 72 | 9136,49 | 7,45 |
| 30 | 4646,43 | 14,15 | 73 | 9249,43 | 26,67 |
| 31 | 4688,05 | 31,78 | 74 | 9291,19 | 35,04 |
| 32 | 4732,37 | 17,33 | 75 | 9375,12 | 58,79 |
| 33 | 4760,03 | 62,14 | 76 | 9464,76 | 35,82 |
| 34 | 4813,43 | 38,33 | 77 | 9519,48 | 89,66 |
| 35 | 4896,95 | 40,58 | 78 | 9621,13 | 71,71 |
| 36 | 4966,02 | 17,88 | 79 | 10102,91 | 69,65 |
| 37 | 5052,1 | 44,86 | 80 | 10189,05 | -0,33 |
| 38 | 5137,16 | 4,66 | 81 | 10262,85 | 2,29 |
| 39 | 5268,85 | 46,78 | 82 | 10617,08 | 29,7 |
| 40 | 5310,04 | 31,23 | 83 | 10701,45 | 1,97 |
| 41 | 5360,04 | 138,98 | 84 | 10843,44 | 40,58 |
| 42 | 5422,73 | 19,14 | 85 | 12392,91 | 2,15 |
| 43 | 5652,14 | 229,61 | 86 | 12503,41 | 9,72 |
| 44 | 5698,21 | 13,9 | 87 | 13441,97 | 3,28 |

La comparaison des spectres peut se faire avec les logiciels connus commercialisés avec les spectromètres tels que BIOTYPER® qui analyse les spectres pics à pics et sont capables de détecter la présence des pics spécifiques d'un spectre de référence en fonction de la valeur d'un score d'identification comme explicité ci-après dans les exemples de réalisation.

Plus particulièrement, on identifie un genre voire une espèce bactérienne contaminante par comparaison avec des spectres de référence de différentes bactéries enregistrées dans la base de données du logiciel BIOTYPER®. Toutefois, pour certaines bactéries et/ou certaines concentrations, comme rapporté dans les exemples ci-après, il n'est pas toujours possible d'obtenir un score d'identification fiable par rapport à des spectres de référence des bases de données de spectres de bactéries enregistrées dans ledit logiciel BIOTYPER®.

C'est pourquoi, dans un mode préféré de réalisation, on compare les spectres selon une nouvelle méthode, à l'étape c), on effectue la comparaison de 2 spectres essentiellement en calculant la soustraction entre les aires totales respectives des 2 spectres, de préférence 2 spectres d'extraits protéiques du même échantillon d'origine après 2 temps d'incubation ou culture différents.

Il s'agit d'une nouvelle méthode d'analyse comparative de spectres avantageuse en ce qu'elle ne considère pas les seuls pics comme le logiciel BIOTYPER® mais la totalité de la courbe du spectre.

On entend ici par «aire totale », la surface entre la courbe du spectre et la ligne de base d'intensité nulle.

Plus particulièrement, qu'à l'étape c), on réalise au moins les étapes successives suivantes :

i.1) on relève les intensités yi de respectivement chacun des points d'abscisse xi correspondant aux valeurs de rapport massique m/z de chaque spectre, et

i.2) pour chaque valeur de xi on calcule une intensité résultante yi3 par soustraction des intensités yi1 et yi2 respectives dudit spectre à analyser (yi1) et respectivement dudit spectre de référence (yi2) :yi3=yi2-yi1, et

i.3) on calcule la somme des intensités résultantes yi3 pour toutes les valeurs xi pour obtenir un score de caractérisation dénommé score différentiel = $\sum_{i=0}^{i=n} |yi3|$.

Plus particulièrement, à l'étape c), on réalise une comparaison des 2 spectres comprenant au moins les étapes suivantes :

i.1) on normalise les intensités yi de respectivement chacun des points d'abscisse xi correspondant aux valeurs de rapport massique m/z de chaque spectre en divisant les intensités yi de chaque point d'abscisse xi (m/z) par l'intensité du pic de plus grande intensité, et

i.2) pour chaque valeur de xi on calcule une intensité résultante normalisée yi3 par soustraction des intensités normalisées respectives de deux spectres yi1 et yi2 :yi3=yi2-yi1, et

i.3) on calcule la somme des intensités résultantes normalisées yi3 pour toutes les valeurs xi pour obtenir un score de caractérisation dénommé score différentiel = $\sum_{i=0}^{i=n} |yi3|$, et de préférence on la normalise en la divisant par la somme des intensités pour toutes les valeurs xi de l'un des spectres, de préférence le spectre de plus petite somme des intensités pour toutes les valeurs xi, pour obtenir un score différentiel normalisé = $\sum_{i=0}^{i=n} |yi3| / \sum_{i=0}^{i=n} yip$ avec p=1 ou 2

De préférence, à l'étape i.3), on réalise les étapes i.3a) et i.3b) de filtrage préalables avant l'étape i.3c) suivantes :

i.3a) pour chaque valeur de xi, on enlève le bruit de fond en donnant la valeur d'intensité résultante normalisée yi3=0 si yi3 est inférieur à un seuil Sy1, Sy1 étant inférieur à 10% de la valeur $yi_{max}$ du pic de plus grande intensité, de préférence Sy1 = 5 % de la valeur $yi_{max}$ du pic de plus grande intensité, et

i.3b) on réalise un filtrage en donnant la valeur yi3=0 pour les valeurs de xi pour lesquels à xi' avec xi'-xi inférieur à un seuil Sx, de préférence Sx étant inférieur ou égal à 10Da, l'intensité résultante normalisée yi3' est de signe opposée à yi3 et de valeur absolue d'au moins une valeur seuil relative de $Sy_2$% de celle de yi3, $Sy_2$ étant de préférence d'au moins 70% de celle de yi3, notamment du fait d'un décalage des valeurs de xi correspondant aux intensités de deux pics d'un même composant des 2 spectres, et

i.3c) on calcule la somme des intensités restantes après filtrage yi3 pour toutes les valeurs xi pour obtenir un score de caractérisation dénommé score différentiel = $\sum_{i=0}^{i=n} |yi3|$, et de préférence on la normalise en la divisant par la somme des intensités pour toutes les valeurs xi de l'un des spectres, de préférence le spectre de plus petite somme des intensités pour toutes les valeurs xi, pour obtenir un score différentiel normalisé = $\sum_{i=0}^{i=n} |yi3| / \sum_{i=0}^{i=n} yip$ avec p=1 ou 2.

On comprend que les valeurs seuil Sx et Sy1 et Sy2 sont des valeurs seuil paramétrables par l'utilisateur. Plus particulièrement, selon le type d'échantillons, on peut paramétrer les valeurs d'intensité et les dites valeurs seuils de manière à ce que l'on détermine au moins l'identité de nature ou de qualité, de l'échantillon à analyser et de l'échantillon de référence comme suit :

- si le score différentiel est inférieur à une valeur v1, v1 étant de préférence de 0.1 à 10, l'identité des deux échantillons est certaine,

- si le score différentiel est compris entre v1 et v2, v2 étant supérieur à v1, de préférence v2 étant égale à 0.1 à 10, l'identité des deux échantillons est possible, et

- si le score différentiel est supérieur à v2, il n'y a pas d'identité des deux échantillons.

Plus particulièrement encore, les différents essais permettent de conclure qu'on peut paramétrer la méthode pour déterminer la présence d'une contamination de l'échantillon si le score différentiel normalisé calculé sur un échantillon incubé pendant un temps t entre 0 et 24H par rapport à un échantillon de référence contaminé par la même bactérie à t=0 ou par rapport à un échantillon de même nature non contaminé dépasse un seuil N1, N1 étant de préférence de 0.1 à 10.

Dans cette méthode, avantageusement, on détermine si l'échantillon d'origine est infecté par une bactérie, si ledit score différentiel calculé à partir des 2 spectres varie, de préférence augmente, entre :

- une première valeur So de score de comparaison entre un premier spectre d'un dit extrait protéique dudit échantillon testé avant culture ou après culture au temps t0 et un deuxième spectre d'un dit extrait protéique dudit échantillon testé respectivement après culture à t1, t1 étant supérieur à t0, et

- au moins une seconde valeur St de score de comparaison entre un premier spectre d'un dit extrait protéique dudit échantillon testé avant culture ou après culture au temps t0 et un deuxième spectre d'un dit extrait protéique dudit échantillon testé respectivement après culture à t2, t2 étant supérieur à t1.

Plus particulièrement encore, on peut paramétrer la méthode pour déterminer la présence d'une contamination de l'échantillon si le ratio St/So entre le score différentiel normalisé St, calculé sur un échantillon incubé pendant un temps t entre 0 et 24H par rapport à un échantillon de référence contaminé par la même bactérie à t=0 ou par rapport à un échantillon de même nature non contaminé, et le score différentiel calculé à t=0 So par rapport à un échantillon de même nature non contaminé dépasse un seuil N2, N2 étant de préférence d'au moins 1.5.

Plus particulièrement encore, on cherche à détecter la présence d'une bactérie choisie au moins parmi les bactéries suivantes : *Escherichia coli, Enterococcus faecalis, Providencia stuartii, Klebsiella pneumoniae, Staphylococcus aureus, Staphylococcus epidermidis, Pseudomonas aeruginosa, Klebsiella oxytoca et Enterobacter cloacae*. Ces espèces bactériennes correspondent à la liste des bactéries impliquées ces 10 dernières années dans la survenue de complications infectieuses après transfusion de CP selon la liste transmise par l'EFS (Etablissement public Français du Sang).

Plus particulièrement encore, à l'étape a.1) on réalise une culture avec un milieu de culture polyvalent, de préférence un milieu liquide contenant essentiellement les composants suivants :

- une source de carbone et d'énergie, généralement le glucose; et

- une source de potassium et de phosphore tel que $K_2HPO_4$ ; et

- une source d'azote et de soufre tel que $(NH_4)_2SO_4$, et

- une source de magnésium tel que $MgCl_2$, et

- une source de calcium tel que $CaCl_2$, et

- une source de fer, de préférence le citrate de fer, et

- une source d'oligo-éléments tels que des sels de Cu, Zn, Co, Ni, B, Ti, et

- de l'eau, de préférence de l'eau distillée.

On comprend que le milieu de culture doit être polyvalent au moins pour les principales bactéries pathogènes listées ci-dessus d'une part et d'autre part compatible avec les autres composants dudit échantillon, notamment qui ne précipite pas en présence de certains composants dudit échantillon, en particulier un milieu ne contenant pas de substance gélifiante et/ou qui coagule en présence de certains composants dudit échantillon, notamment en présence de plaquettes, et compatible avec le traitement ultérieur de lyse cellulaire et d'extraction protéique et la réalisation d'un dit spectre de masse. Des composants gélifiants tels que le fibrinogène sont incompatibles.

Pour détecter une bactérie ou levure particulière, notamment une bactérie ou levure pathogène, on mettra en oeuvre un milieu de culture et des conditions de culture optimales pour ladite bactérie ou levure favorisant sa croissance dans ledit échantillon d'origine. Plus particulièrement, ledit milieu de culture est un milieu acellulaire conventionnel de bactérie, de préférence un milieu comprenant des composant choisis parmi un extrait de broyat ou lysat de tissu pluricellulaire, un digestat enzymatique, notamment un digestat enzymatique de caséine, soja et/ou de tissu animal, une peptone, un extrait de levure, un sucre tel que dextrose ou glucose, un sel tel que NaCl et/ou Na2PO4.

Plus particulièrement encore, on détermine si l'échantillon de produit sanguin, de préférence un concentré de plaquettes, est infecté par une bactérie, de préférence à une concentration d'au moins 10 bactéries/ml, si ledit score différentiel calculé à partir des 2 spectres augmente entre :

- une première valeur de score de comparaison entre un premier spectre d'un dit extrait protéique dudit échantillon testé avant culture ou après culture au temps t0 et un deuxième spectre d'un dit extrait protéique dudit échantillon testé respectivement après culture à t1, t1 étant supérieur à t0, de préférence to étant de 0 à 6h et t1 étant de 2 à 24h, de préférence encore to étant de 0 à 2h et t1 étant de 2 à 6h, et

- au moins une seconde valeur de score de comparaison entre un premier spectre d'un dit extrait protéique dudit échantillon testé avant culture ou après culture au temps t0 et un deuxième spectre d'un dit extrait protéique dudit échantillon testé respectivement après culture à t2, t2 étant supérieur à t1, de préférence to étant de 0 à 2h , t1 étant de 2 à 6h et t2 étant de 4 à 16h.

Plus particulièrement, on peut mettre en oeuvre un programme d'ordinateur comportant les instructions pour l'exécution des étapes i.1) à i.3) du procédé de traitement de spectres, de préférence un support d'enregistrement lisible par un ordinateur sur lequel est enregistré ledit programme selon l'invention.

D'autres caractéristiques et avantages de la présente invention ressortiront mieux à la lecture de la description qui va suivre, faite de manière illustrative et non limitative, en référence aux dessins annexés sur lesquels :

Les figures 1 et 2 illustrent un spectre de masse caractéristique d'extrait protéique d'échantillon standard de concentrés de plaquettes non contaminés comme commenté aux exemples 2 et3.

Les figures 3A à 3C représentent les 2 spectres de concentrés de plaquettes contaminé par *E.coli* et respectivement non contaminé analysés et comparés avec le nouveau logiciel dénommé logiciel IHU décrit à l'exemple 5.

Les figures 4A à 4C représentent les 2 spectres de concentrés de plaquettes contaminé par *S.Aureus* et non contaminé, analysés et comparés avec le nouveau logiciel dénommé logiciel IHU décrit à l'exemple 5.

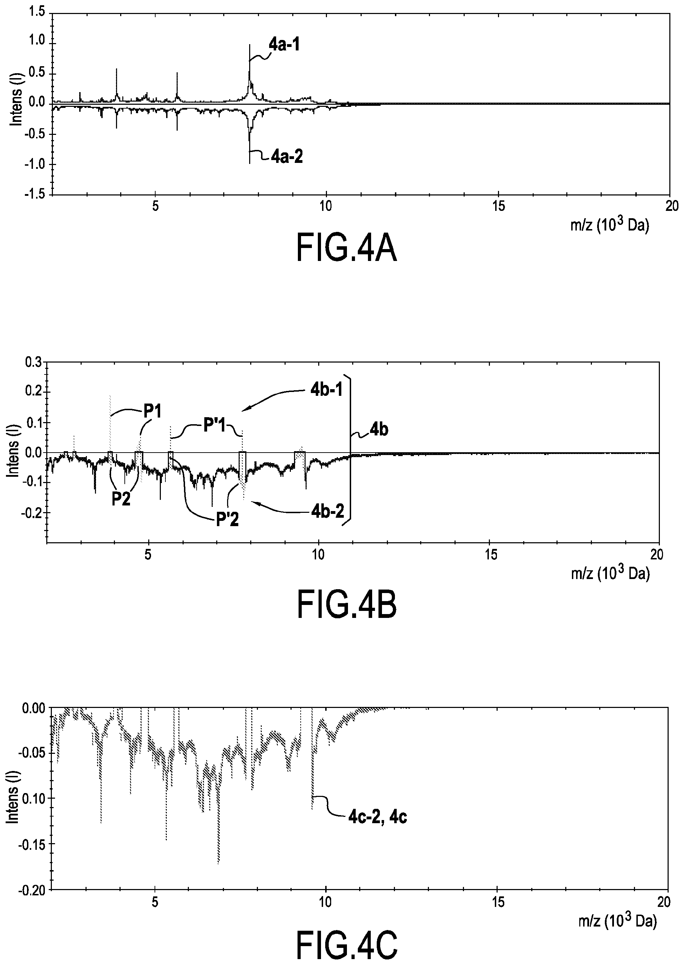

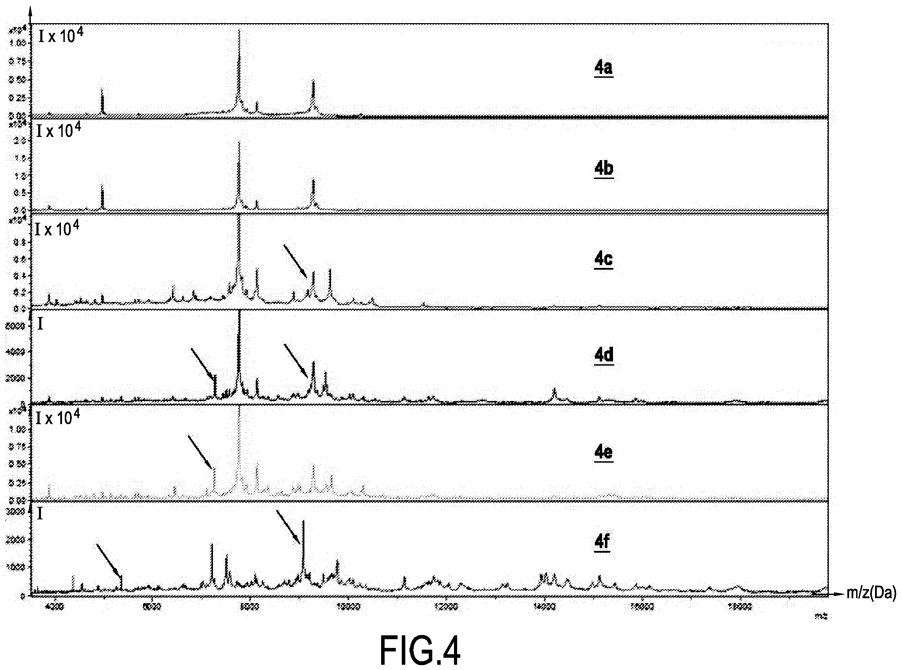

La figure 4 représente différents spectres d'extrait protéique de concentré de plaquettes, non contaminé (spectres 4a et 4b), contaminés par différentes bactéries *S. aureus (4c), E. coli* (4d), *P. stuartii* (4e), *P. aeruginosa* (4f) discuté à l'exemple 6.

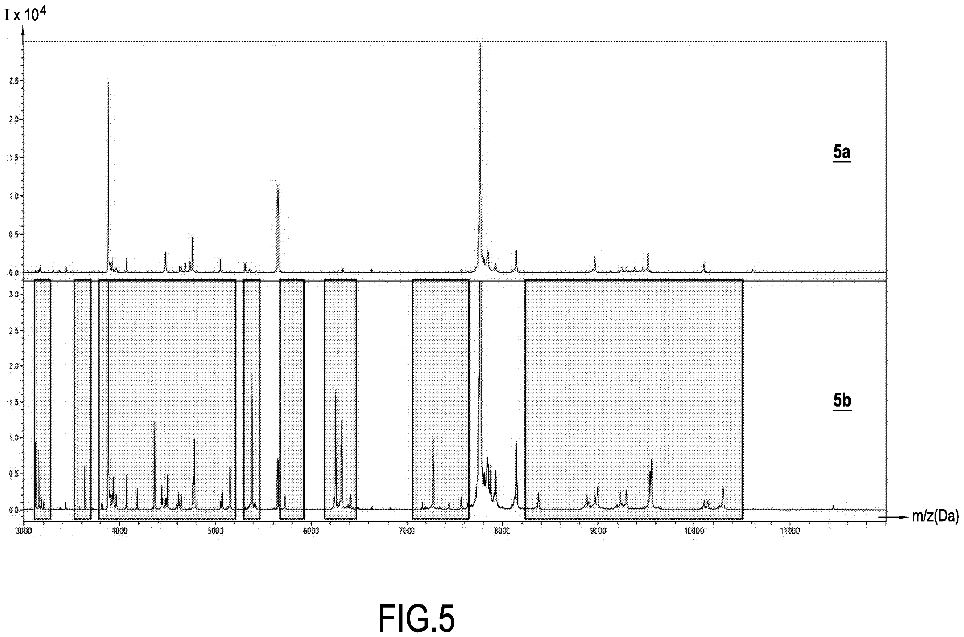

La figure 5 montre 2 spectres d'extrait protéiques de concentré de plaquettes non contaminés (spectre 5a) et contaminé (spectre 5b) par la bactérie *E. coli* après une incubation de 6h sans milieu de culture commenté à l'exemple 7.

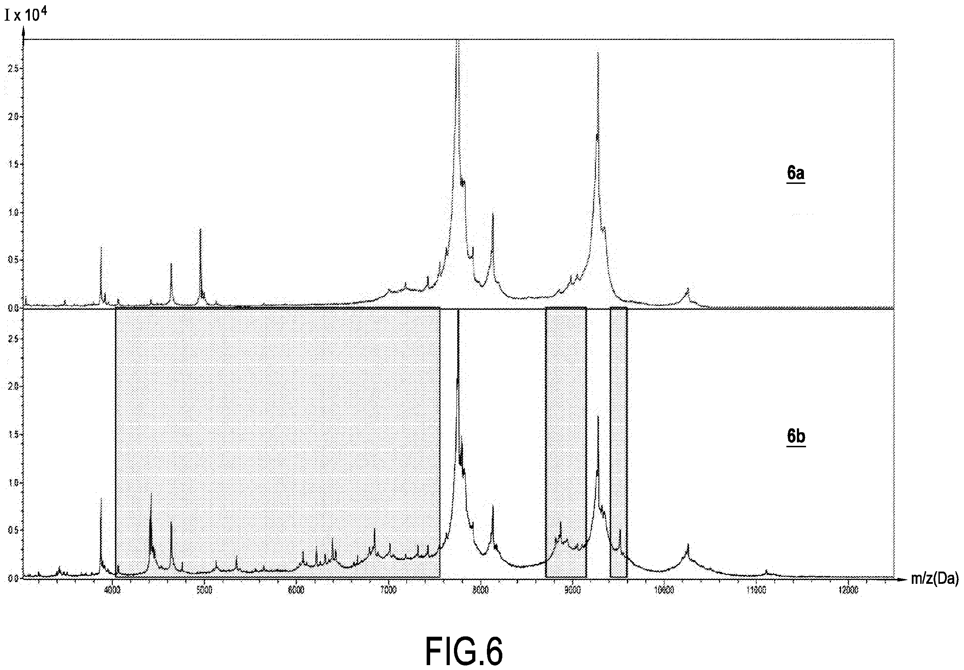

La figure 6 montre 2 spectres d'extraits protéiques de concentré de plaquettes non contaminées (spectre 6a) et contaminé (spectre 6b) par la bactérie *Enterococcus faecalis* en présence de milieu de culture commenté à l'exemple 8.

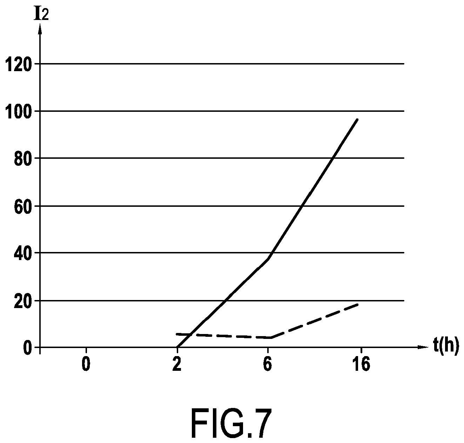

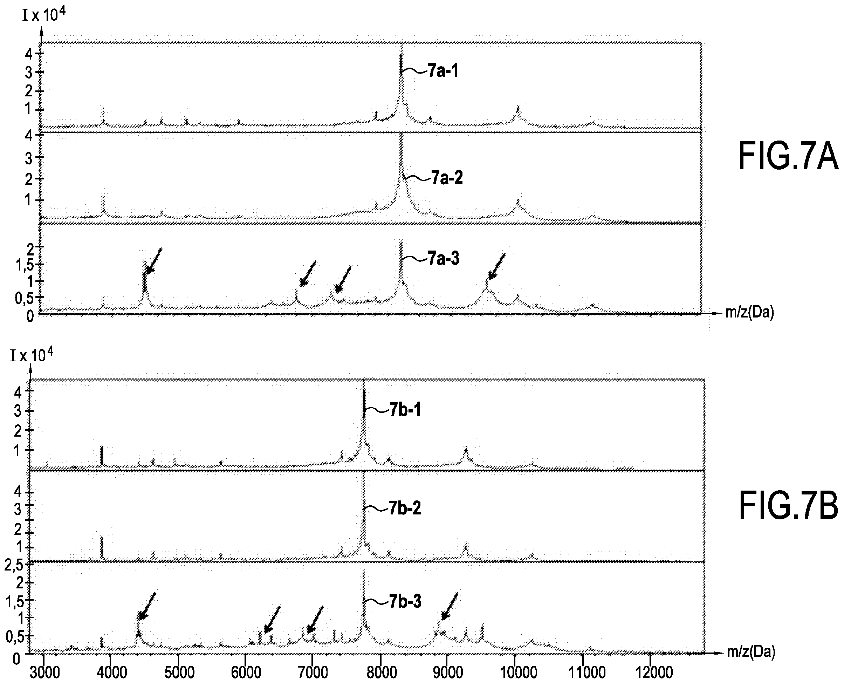

La figure 7 montre l'évolution du score 12 (score différentiel normalisé) pour un échantillon de concentré de plaquettes contaminé par *K.pneumoniae* avec milieu de culture (courbe en gras) et sans milieu de culture (courbe en tirets espacés) commenté à l'exemple 9.

Les figures 7A et 7B montrent des spectres obtenus pour des concentrés de plaquettes avant incubation (spectres 7a-1 et 7b-1) et après incubation en absence de milieu de culture (spectres 7a-2 et 7b-2) et en présence de milieu de culture (spectres 7a-3 et 7b-3), pour des concentrations de bactéries *Klebsiella pneumoniae* de $10^7$ ufc/ml (figure 7A) et $10^2$ ufc/ml (figure 7B) commentées à l'exemple 9.

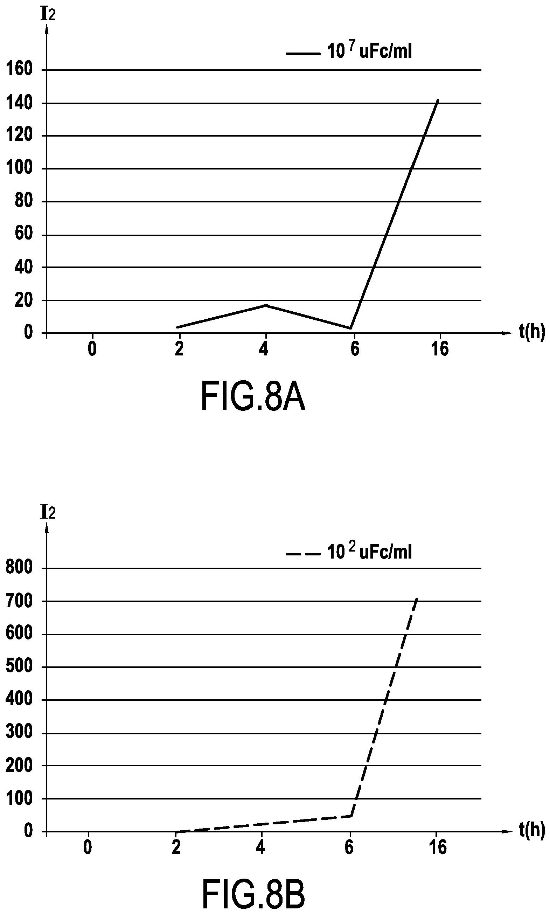

Les figures 8A et 8B représentent les courbes d'évolution du score différentiel normalisé 12 pour des concentrations de $10^7$ufc/ml (figure 8A) et respectivement $10^2$ufcC/ml (figure 8B) pour des concentrés de plaquettes contaminée par *K. pneumoniae* avec milieu de culture (courbe en gras) et sans milieu de culture (courbe en tirets espacés) commentées à l'exemple 9.

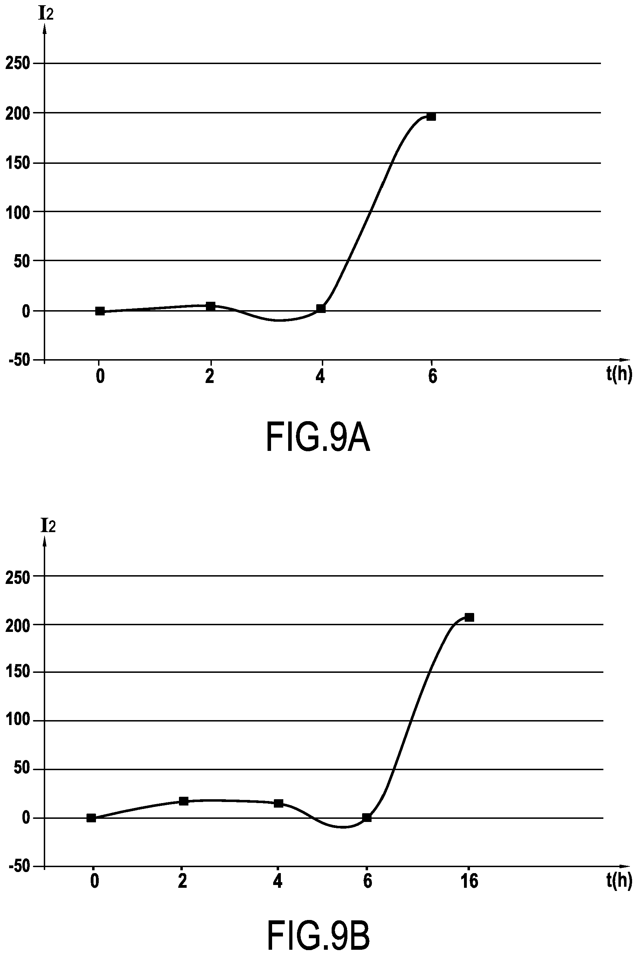

Les figures 9A et 9B représentent des courbes d'évolution du score différentiel 12 non normalisé pour *E.coli* sans milieu de culture de 0 à 6h (figure 9A) et avec milieu de culture de 0 à 16 h (figure 9B) commentées à l'exemple 9.

**Exemple 1:** Méthode d'acquisition de spectres de masse de type maldi-tof.

On a tenté de réaliser un spectre de concentré plaquettaire brut en suivant le protocole conventionnel suivant sans succès.

Sur une plaque d'un appareil spectromètre Microflex LT de Bruker Daltonics™, on dépose 1 $\mu$l de concentré plaquettaire puis 1 $\mu$l de solution de matrice HCCA décrite ci-après et on laisse sécher à température ambiante.

Le concentré plaquettaire était obtenu à partir d'une poche d'échantillonnage de CP stérile apyrogène de 30 ml de la société MacoPharma REF: VSE 0000A.

Le spectre est acquis en mode automatique sur un Microflex LT en utilisant la méthode suivante décrite ci-après. Aucun spectre exploitable n'a pu être obtenu par le logiciel BIOTYPER® ou le nouveau logiciel décrit ci-après

1. La préparation de la solution de matrice (HCCA saturée) comprend les étapes suivantes consistant à :.

- introduire dans un tube polypropylène de 1.5 ml, 2 pointes de spatules d'acide alpha-cyano-4-hydroxycinnamique (HCCA),

- ajouter 500 $\mu$l d'acétonitrile HPLC, 475 $\mu$l d'eau HPLC, 25 $\mu$l de TFA (acide trifluoroacetique),

- mélanger au Vortex ou secouer vigoureusement,

- soniquer 10 min puis centrifuger 5 min à 13000 g,

- transférer le surnageant dans un tube polypropylène 1.5 ml propre.

2. La méthode d'acquisition des spectres comprend les étapes et caractéristiques suivantes :

- introduire la plaque (cible Microflex modèle de référence 224989 dénommé MSP 96) contenant les dépôts dans le spectromètre de masse Microflex LT,

- démarrer l'ordinateur associé au spectromètre pour lancer les analyses, et

- lancer l'acquisition des spectres de masse MALDI-TOF.

Les paramètres appliqués au spectromètre de masse sont les suivants :

- mode linéaire positif, Electrode IS1 : 20,00 kV, Electrode IS2 : 18,05 kV, Electrode de focalisation : 6 kV, Fréquence du laser : 60Hz, Gain du détecteur : 8,8 X, Post-ion-extraction (PIE) : 120 ns, Gamme de masse : de 700 à 20000 Da.

Les spectres sont obtenus via une méthode automatique contrôlée par le logiciel FLEXCONTROL® du fabricant BRUKER DALTONICS® les paramètres sont les suivants pour chaque spot :

- nombres séries de 100tirs: 20 sur des positions différentes du spot (20X1000 = 2000 spectres)

- afin que les tirs aient balayé l'ensemble de l'échantillon de façon homogène l'acquisition se fait sur 6 positions différentes selon une géométrie hexagonale.

- pré-tirs : 10 tirs à 40% de la puissance maximum laser servent à dessaler l'échantillon (contamination par les sels minéraux)

- puissance laser : 30 % à 45 % régulée via le logiciel FLEXCONTROL®

- sélection du spectre : sur une gamme de masse de 4 000 à 10 000 Da.

L'acquisition des spectres est au final réalisée via le logiciel BIOTYPER® du fabricant BRUKER DALTONICS® qui permet d'appliquer la méthode automatique décrite ci-dessus à une série d'échantillons en prenant en compte les critères mentionnés précédemment et un score de qualité ou score de validation qui représentent l'addition de 2 notes ni + n2 (ni étant fonction du nombre de pics et n2 fonction du rapport signal/bruit de fond).

- ni est la note attribuée en fonction du nombre de pics caractéristiques du spectre n, avec : ni = 0 pour $n \leq 8$, ni = 1 pour $8 < n \leq 27$, n1 = 2 pour $27 < n \leq 43$, n1 = 3 pour $43 < n \leq 78$, n1 = 4 pour $78 < n \leq 86$, n1 = 5 pour $86 < n \leq 110$, n1 = 6 pour $110 < n$, et

- n2 est la note attribuée en fonction de la valeur du rapport signal/bruit (Rmax) du pic de plus grande intensité du spectre, avec n2 = 0 pour $Rmax \leq 6,8$, n2 = 1 pour $6,8 < Rmax \leq 26,8$, n2 = 2 pour $26,8 < Rmax \leq 52,6$, n2 = 3 pour $52,6 < Rmax \leq 208,6$, n2 = 4 pour $208,2 < Rmax \leq 398,6$, n2 = 5 pour $398,6 < Rmax \leq 1\,092,2$, n2 = 6 pour $1\,092,2 < Rmax$.

Un spectre est pris en compte si son score de validation (n1+n2) est supérieur à 2.

**Exemple 2 :** Protocole de traitement et préparation d'un extrait protéique d'un concentré plaquettaire.

1) Après de multiples essais avec différents réactifs et traitements divers, le protocole suivant a permis d'obtenir des spectres de spectrométrie de masse maldi-tof selon la méthode ci-dessus décrite.
Le protocole de traitement et préparation de un extrait protéique de lysat cellulaire de CP comprend les étapes successive suivantes consistant à :

- mettre 1 ml de concentré plaquettaire dans un tube eppendorf, et le centrifuger à 14000G pendant 5 min. puis éliminer le surnageant, et

- laver le culot avec 1 ml d'eau, après avoir centrifugé 5 min à 14000G, puis éliminer le surnageant, et

- ajouter 900 $\mu$l d'eau et 300 $\mu$l de solution de saponine (0.5g/ml dans de l'eau) dans le culot de centrifugation, puis mélanger dans un Vortex, puis centrifuger à 14000G pendant 5 min, puis éliminer le surnagent, et

- laver le culot avec 1 ml d'eau, après avoir centrifugé 5 min à 14000G, puis éliminer le surnageant, puis

- ajouter 25 $\mu$l d'une solution d'acide formique à 70% ( 700microlitre d'acide formique dans 300 microlitre d'eau ) dans le culot de centrifugation , puis mélanger par mouvement aller et retour de bas en haut avec une micropipette Eppendorf , puis rajouter encore 25 $\mu$l d'acétonitrile pur et mélanger par mouvement de up et down avec la micropipette, puis centrifuger à 14000G pendant 5 min, puis

- récupérer la solution claire de surnageant correspondant à un d'extrait protéique de lysat cellulaire de l'échantillon de CP d'origine.

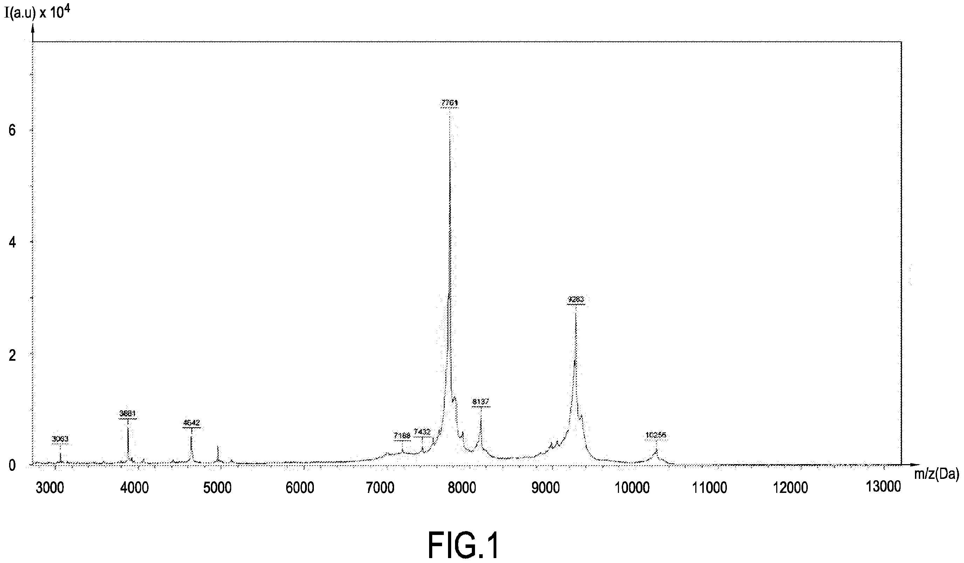

La centrifugeuse était une centrifugeuse HERAEUS, référence Biofuge pico de la société THERMOSCIENTIFIC[®].
2) Des spectres ont été obtenus comme montré à la figure 1 après dépose de 1 $\mu$l de la solution claire sur le cible MALDI-TOF (MSP 96, Bruker Daltonics référence 224989), puis séchage à température ambiante, puis dépose de 1 $\mu$l de solution de matrice et à nouveau séchage à température ambiante. Le spectre est acquis en mode automatique sur un Microflex LT en utilisant la méthode décrite précédemment.

Le spectre de la figure 1 présente la liste des pics rapportés dans le tableau 1 mentionné ci-dessus.

Les échantillons de CP ont été traités par l'étape d'extraction selon la méthode décrite ci-dessus. La figure 1 est un exemple représentatif de spectres obtenus sur des extraits de 35 poches de CP d'individus de groupes sanguins variables. On observe une reproductibilité des spectres inter-poches (n=35) de concentrés de plaquettes non infectées quelques soit le groupe sanguin.

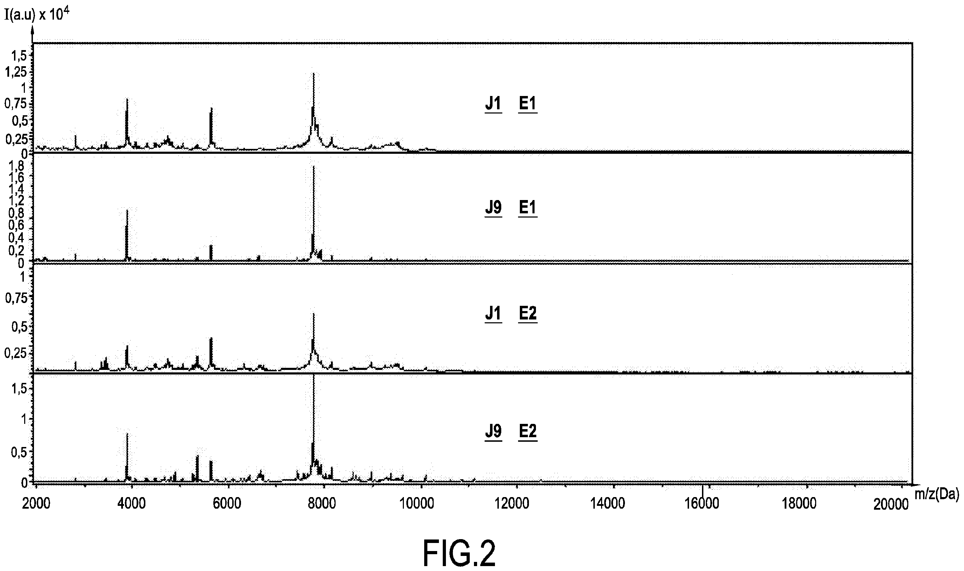

Les concentrés de plaquettes ont été conservés pendant 9 jours. Chaque jour, les échantillons ont été traités par l'étape d'extraction Acide formique/Acétonitrile selon la méthode décrite ci-dessus. Les spectres d'extrait protéique de CP obtenus le premier jour (J1) et le neuvième jour (J9) ont été comparés. La figure 2 illustre la reproductibilité des spectres intra-poches au cours du temps pour 2 échantillons de CP E1 et EV2. Le spectre des plaquettes à J9 est sensiblement identique au spectre des plaquettes à J1.

Exemple 3 : Préparation d'extrait protéique d'échantillon de concentrés plaquettaires contaminés par des bactéries.

1) Préparation des inocula bactériens :
On a réalisé le protocole comprenant les étapes successives suivantes consistant à :

- mettre en culture le germe d'intérêt sur une gélose au sang, puis

- après 24 heures de culture, prélever l'ensemble des colonies afin de préparer une solution d'inoculum dans 15 ml d'eau stérile, puis

- verser un aliquote de1 ml dans 15 tubes eppendorfs, les diluer au 10eme en cascade, puis

- déposer 100 $\mu$l des différentes dilutions sur une gélose au sang et étaler de façon homogène à l'aide d'un râteau d'étalement, puis

- laisser incuber 24H à 37°C, puis compter les colonies apparues sur une dilution donnant de 100 à 200 colonies.

La concentration de l'inoculum obtenu en UFC /ml est le nombre de colonies X (1/la dilution) X10UFC : unité formant colonies.

2) Préparation de concentré plaquettaire contaminé.
On a réalisé le protocole comprenant les étapes successives suivantes consistant à :

- prélever 900 $\mu$l de concentré plaquettaire à partir de la poche et les mettre dans un tube eppendorf de 2 ml, puis ajouter 100 $\mu$l de solution bactérienne ci-dessus à des concentrations de $10^2$ à $10^8$ ufc/ml (soit en concentration finale de $10^1$ à $10^7$ ufc/ml), puis

- incuber les tubes de 0 à 24 heures à 37°C.

Ensuite on réalise le même traitement et préparation d'extrait protéique que décrit ci-dessus consistant à :

- à chaque temps, prendre le tube eppendorf de 2ml, le centrifuger à 14000G pendant 5 min et éliminer le surnageant, et

- ajouter 900 $\mu$l d'eau et 300 $\mu$l de solution de saponine, vortexer puis centrifuger à 14000G pendant 5 min et éliminer le surnageant, et

- laver avec 1 ml d'eau, après avoir centrifugé 5 min à 14000G, éliminer le surnageant, puis

- ajouter 25 µl d'acide formique à 70%, mélanger par mouvement avec la micropipette, puis rajouter 25 µl d'acétonitrile et mélanger par mouvement avec la micropipette, puis centrifuger à 14000G pendant 5 min, puis

- déposer 1 µl de la solution claire de surnageant sur la cible MALDI-TOF, laisser sécher à température ambiante. Déposer 1 µl de solution de matrice et laisser sécher à température ambiante. Le spectre est acquis en mode automatique sur un Microflex LT en utilisant la méthode décrite précédemment à l'exemple 1.

3) Protocole d'incubation avec un milieu de culture des concentrés de plaquettes contaminés.

On a réalisé le protocole comprenant les étapes successives suivantes consistant à :

- prélever 900 µl de concentré plaquettaire à partir de la poche et les mettre dans un tube eppendorf de 2ml, puis ajouter 100 µl de solution bactérienne à des concentrations de $10^2$ à $10^8$ (soit en concentration finale de $10^1$ à $10^7$), puis

- ajouter 1 ml de milieu de culture dans chaque tube, et

- incuber de 0 à 24 heures à 37°C.

Des essais ont été réalisés avec les 3 milieux de cultures différents suivants comme décrit aux exemples 6 à 8 ci-après.

a) Milieu coeur-cervelle présentait la composition suivante pour 1 litre :

| | |
|---|---|
| - Infusion solide de cervelle | 12.5 g |
| - Infusion solide de coeur de boeuf | 5 g |
| - Proteose peptone | 10 g |
| - Glucose | 2 g |
| - NaCl | 5g |
| - Phosphate disodique | 2.5 g |

b) Milieu thiosulfate de sodium de composition suivante pour 1 litre :

| | |
|---|---|
| peptone de caséine (bovine) : | 15 g |
| L-cystine : | 0.5 g |
| glucose anhydre : | 5 g |
| extrait de levure : | 5 g |
| chlorure de sodium : | 2.5 g |
| thioglycolate de sodium : | 0.5 g |
| résazine : | 0.001 g |
| agar : | 0.75 g |
| eau : | 1l |

c) Milieu trypticase Soja de composition suivante :

| Formule approximative par litre d'eau purifiée | |
|---|---|
| Digestion pancréatique de caséine | 17,0 g |
| Digestion papaïque de semoule de soja | 3,0 g |
| Chlorure de sodium | 5,0 g |
| Phosphate bipotassique | 2,5 g |
| Dextrose | 2,5 g |

Ensuite, on a réalisé le même traitement et préparation d'extrait protéique et même protocole d'acquisition de spectre par spectrométrie de masse maldi-tof avec le même appareillage Microflex que décrit ci-dessus.

4) Des poches de concentrés plaquettaires contaminés ont été réalisées selon le protocole comprenant les étapes successives suivantes consistant à :

- prélever 2 fois 1 ml de concentré plaquettaire CP (qui serviront de témoins négatifs), puis

- ensemencer d'une dilution bactérienne de 1 ml à une concentration prédéfinie dans de l'eau dans un tube eppendorf de 2 ml, par injection avec une seringue dans une tubulure du MCP au plus près de l'entrée de la poche, puis

- réaliser une soudure de la tubulure avec une soudeuse automatique en amont du point d'injection, puis

- remuer le CP pour obtenir une répartition homogène des germes.

L'échantillonnage est réalisé à différents temps via l'utilisation d'une connexion de la tubulure du CP. Une fois le volume désiré récupéré dans une poche d'échantillonnage et une soudure de la tubulure du CP est réalisée.

Le suivi de la contamination des poches de concentré plaquettaires comprenant les étapes successives suivantes consistant à :

- après la contamination, prélever 3 échantillons de volumes 1ml, 8ml et 16ml et appliquer le protocole d'incubation dans les plaquettes+milieu de culture décrit paragraphe 3) ci-dessus, puis

- après 24 heures incubation à 24°C sous agitation (protocole standard de conservation des concentrés de plaquettes) prélever 3 échantillons de volumes 1ml, 8ml et 16ml et appliquer le protocole d'incubation dans les plaquettes+milieu de culture décrit paragraphe 3) ci-dessus.

La comparaison de 2 spectres a été réalisée par le logiciel Biotype® r3.0 et par un nouveau logiciel exécutant nouvel algorithme fourni par la présente invention comme décrit aux exemples 4 et 5 ci-après.

**Exemple 4 :** Analyse des spectres de masse pour la détection de contamination bactérienne par le logiciel BIOTYPER®.

Le logiciel BIOTYPER® fourni le fabricant du spectromètre de masse MALDI-TOF (Bruker Daltonics) par permet de comparer un nouveau spectre avec les spectres moyens contenus dans la base de données. Le logiciel BIOTYPER® identifie des spectres inconnus par comparaison avec des spectres de référence stockés dans une base de données ou autre spectre comparatif par une analyse des pics qu'ils contiennent. Les résultats d'identification sont donnés sous forme de d'un score d'identification calculé comme décrit dans le manuel d'utilisation du logiciel et résumé ci-après.

Dans un premier temps, les deux spectres sont alignés. On assigne une erreur acceptable pour la masse des pics de chaque spectre correspondant à 800 ppm ($\pm$1 Da) et le logiciel repositionne les deux spectres (autrement dit, si l'écart entre les pics du même spectre et entre les pics de l'autre spectre est trop différent, le logiciel ne pourra pas aligner les spectres).

Ensuite, les spectres obtenus subissent le traitement suivant par le logiciel BIOTYPER® 3.0 :

- une soustraction de ligne de base, afin d'éliminer le bruit de de fonds, et

- un lissage des pics par mise à 0 des intensités inférieures à un seuil donné, afin d'augmenter la *résolution*, puis

- une liste de masse de pics est créée.

En résumé, le score d'identification est égal à log (indice1 + incide2 + indice3), avec :

- un indice 1 de valeur maximale de 1000 quand l'ensemble des pics de la référence de la base de données sont trouvés dans le spectre à identifier, l'indice 1 étant de zéro si il n'y a aucune correspondance, et

- un indice 2 étant de valeur maximale de 1000, si l'ordre de classification des pics par intensité croissante du spectre à identifier est identique à l'ordre de classification des pics de la référence, et

- un indice 3 étant de valeur maximale de 1000, si les pics non attribués du spectre à identifier ne matche sur aucune référence.

Ainsi, un score d'identification compris entre 0 et 1,699 permet d'exclure l'identité du spectre de l'échantillon testé avec celui du spectre de référence. Un score d'identification compris entre 1,700 et 1,999 suggère que l'échantillon testé appartient ou comprend l'élément de référence et un score d'identification supérieur à 2,0 permet une identification certaine de l'échantillon testé celui du spectre de référencer présent dans la base de données.

Pour une bactérie, l'identification est la suivante :

- de 0 à 1,69 : identification non fiable

- de 1,7 à 1,99.0 : identification possible, notamment du genre pour une bactérie

- de 2.0 à 3.0 : identification certaine, notamment du genre et de l'espèce pour une bactérie.

**Exemple 5 :** Nouveau logiciel exécutant un nouvel algorithme de comparaison de 2 spectres selon la présente invention.

1) Le principe du logiciel IHU est original en ce qu'il ne réalise pas une étude par comparaison des seuls pics mais prend en compte la totalité de la courbe des spectres. Le logiciel développé travaille à partir de 2 spectres de spectrométrie de masse et comprend les étapes suivantes :

- les 2 spectres sont représentés selon un axe des abscisses des masses, les intensités I exprimées en unité arbitraire et sont représentées en sens inverse avec le spectre de référence (concentré de plaquettes non contaminé) au-dessus de l'axe des abscisses et le spectre de l'échantillon à analyser (concentré de plaquettes contaminé) en dessous. Ainsi, les alignements de pics relatifs au même composant ou décalages d'alignement éventuels des pics des 2 spectres apparaissent plus clairement. Un décalage de pic peut résulter d'une erreur pour des pics relatifs à un même composant et qui auraient dû être soustraits si le décalage n'excède pas 100Da ou le décalage peut au contraire correspondre à 2 pics relatifs à des composants différents.

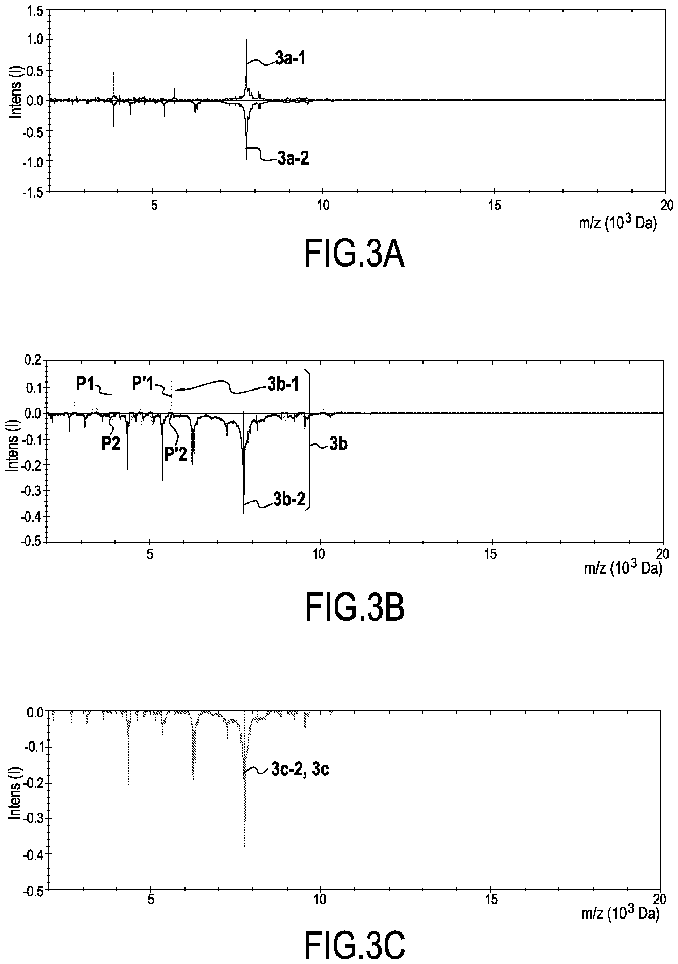

- dans une première étape (figures 3A et 4A), les deux spectres, spectre 1 (spectre de référence) et spectre 2 (spectre de l'échantillon à analyser), à comparer sont mis à l'échelle (en se basant sur l'intensité du pic de plus grande intensité), c'est-à-dire que pour chaque spectre les intensités y1 en ordonnée pour chaque valeur de x1 en abscisse (m/z) sont normalisées en divisant yi par l'intensité du pic de plus grande intensité du spectre. - Dans une deuxième étape (figures 3B et 4B), les deux spectres sont ensuite soustraits pour chaque valeur de xi, on calcule yi3=yi2-yi1 pour obtenir un spectre 3 = spectre 1-spectre 2, et

- dans une troisième étape (figure 3C et 4C), le bruit de fond du spectre différence est enlevé en mettant à 0 les valeurs yi3 inférieur à un certain seuil, par exemple inférieur à Sy1=5% du pic de plus grande intensité tel que décrit ci-dessus en liaison avec l'étape i.3a, et on filtre par mise à 0 les valeurs de yi3 correspondant à des pics qui ne sont pas bien alignés entre les 2 spectres et n'ont pu être soustraits correctement tel que décrit ci-dessus en liaison avec l'étape i.3b avec une valeur Sy2 de 70% et Sx= (100Da), et

- à partir de ce spectre résultant après filtrage ci-dessus, on calcule un score différentiel normalisé qui est l'aire total résiduel du spectre (positive et négative) divisé par l'intensité total du premier spectre selon la formule

$$\sum_{i=0}^{i=n} |yi3| / \sum_{i=0}^{i=n} yi1.$$

A titre illustratif, les figures 3A à 3C représentent les 2 spectres analysés et comparés avec le nouveau logiciel IHU. La figure 3A montre les 2 spectres bruts 3a-1 et 3a-2 obtenus pour des extraits protéiques obtenus à partir d'échantillons de concentré de plaquettes contaminées par *E. coli* respectivement à t = 0 (spectre 3a-1) figure et t = 6h (spectre 3a-2). Les 2 spectres sont représentés selon un axe des abscisses des masses, les intensités I exprimées en unité arbitraire et sont représentées en sens inverse de sorte que l'alignement des pics relatifs au même composant ou décalage d'alignement éventuel des pics des 2 spectres apparaisse plus clairement. Un décalage de pic peut résulter d'une erreur pour des pics relatifs à un même composant si le décalage n'excède pas une valeur paramétrable par l'utilisateur en général 10Da ou au contraire correspondre à 2 pics relatifs à des composants différents.

Sur la figure 3A, le spectre 3a-1 est le spectre initial d'un extrait protéique selon l'invention de concentré plaquettaire contaminé par E.coli à $10^3$/ml bactéries à to (avant incubation) et le spectre 3a-2 est le spectre brut initial d'un même extrait protéique selon l'invention mais à t=6h après incubation. A ce stade , pour le spectre 1 (3a-1) la somme

$\sum_{i=0}^{i=n} yi1 = 67.424$ =67.424 et, pour le spectre 2 (3a-2) la somme $\sum_{i=0}^{i=n} yi2 = 796.134$.

Sur la figure 3B, on a représenté le spectre 3a-3 de soustraction des deux spectres 1 (3a-1) et 2 (3a-2) après leur normalisation. On voit pour certaines valeurs de xi et xi' avec xi'-xi inférieur à 10Da, il y a des changements de signes des intensités résultantes normalisées yi3=yi2-yi1 et yi3'=yi2'-yi1'. Ainsi, pour les pics p1/p2, et p'1/p'2, il s'agit de décalages résultant d'une erreur pour des pics d'un même composant résultant d'une erreur de décalage. A ce stade, pour le spectre 1 (3a-1) la somme $\sum_{i=0}^{i=n} yi1 = 5.981$ et pour le spectre 2 (3a-2) la somme $\sum_{i=0}^{i=n} yi2$ =734.869.

Sur la figure 3C, on a représenté le spectre 3a-4 de soustraction des deux spectres 1 (3a-1) et 2 (3a-2) après leur normalisation puis le filtrage des pics mal soustraits du fait d'un décalage de la figure 3B. Sur la figure 3C, il n'y a plus de valeurs de xi avec des changements de signes des intensités résultantes entre xi et xi' pour xi'-xi inférieur à 10Da. A ce stade, pour le spectre 1 (3a-1) la somme $\sum_{i=0}^{i=n} yi1 = 1.072$ et pour le spectre 2 (3a-2) la somme $\sum_{i=0}^{i=n} yi2 = 583.727$ et le score différentiel normalisé est de $\sum_{i=0}^{i=n} |yi3| / \sum_{i=0}^{i=n} yi1 = 8.673$.

2) A titre illustratif, les figures 3A à 3C et 4A à 4C représentent les spectres des échantillons suivants analysés et comparés avec le nouveau logiciel IHU.

Les figures 3A et 4A montrent les spectres bruts obtenus pour des extraits protéiques obtenus à partir d'échantillons de concentré de plaquettes contaminées par $10^7$ E. coli par ml (3a-2, figure 3A) et par $10^7$ S.aureus par ml (4a-1 figure 4A), ainsi que les spectres des mêmes concentrés plaquettaires non contaminés (3a-1, figure 3A et 4a-1 figure 4A).

Sur les figures 3B et 4B, on a représenté les spectres 3b et 4b de soustraction des deux spectres 1 (3a-1 / 4a-1) et 2 (3a-2 / 4a-2) après leur normalisation. Sur les figures 3C et 4C, on a représenté les spectres 3c et +4c de soustraction après élimination du bruit de fond et après le filtrage des pics mal soustraits des spectres 3b et 4b des figures 3B et 4B.

Sur les figures 3B-3C et 4B-4C, des pics résiduels en positifs 3b-1 et respectivement 4b-1, proviennent de concentré de plaquettes non contaminé et les pics résiduels en négatifs 3b-2, 4c-2 et respectivement 4b-2,4c-2 proviennent de concentré de plaquettes contaminé. Sur les figures 3C et 4C, il n'y a plus de pics résiduels en positifs.

Sur les figures 3B et4B, on voit pour certaines valeurs de xi et xi' avec xi'-xi inférieur à 100Da, il y a des paires de pics alignées p1/p2 et p1'/p2' avec changements de signes des intensités résultantes yi3=yi2-yi1 et yi3'=yi2'-yi1'. Sur les figures 3C et 4C, il n'y a plus de valeurs de xi avec des changements de signes des intensités résultantes entre xi et xi' pour xi'-xi inférieur à 100Da.

Les pics de couleur plus claire des figures 3B et 4B sont les pics qui sont éliminés et filtrés sur les figures 3C et 4C.

Sur les figure 3A et 4A, pour le spectre 1 (3a-1 et 4a-1) la somme $\sum_{i=0}^{i=n} yi1 = 247.741$ (figure 3A) et 623,235 (figure 4A), pour le spectre 2 (3a-2 et 4a-2) la somme $\sum_{i=0}^{i=n} yi2 = 495,99$ (figure 3A) et 14231,294 (figure 4A).

Figures 3B et 4B, pour le spectre 1 (3b-1 et 4b-1) la somme $\sum_{i=0}^{i=n} yi1 = 0.006$ (figure 3B) et 0 (figure 4B), et pour le spectre 2 (3b-2 et 4b-2) la somme $\sum_{i=0}^{i=n} yi2 = 222.533$ (figure 3B) et 579,794 (figure 4B), et le score différentiel normalisé avant filtrage est de $\sum_{i=0}^{i=n} |yi3| / \sum_{i=0}^{i=n} yi1 = 0,898$ (figure 3B) et 0,917(figure 4B).

Sur les figures 3C et 4C, pour les spectres 1 (3c-1 et 4c-1) la somme $\sum_{i=0}^{i=n} yi1 = 0$, et pour les spectres 2 la somme $\sum_{i=0}^{i=n} yi2 = 137.901$ (figure 3C) et 430,243 (figure 4C),et le score différentiel normalisé après filtrage (score IHU définitif) est de $\sum_{i=0}^{i=n} |yi3| / \sum_{i=0}^{i=n} yi1 = 0.557$ (figure 3C) et 0,681 (figure 4C).

Les différents essais permettent de conclure que pour ce type d'échantillons de concentrés de plaquettes contaminés par des bactéries, avec les paramétrages des différentes valeurs seuils mentionnés ci-dessus, l'on détermine ici la présence ou l'absence d'une contamination de l'échantillon si le score différentiel normalisé calculé sur un échantillon contaminé à t=0 par rapport à un échantillon de référence non contaminé comme suit:

- si le score différentiel est inférieur à une valeur v1, v1 étant de par exemple notamment égale à 0.2, l'identité des deux échantillons est certaine, et donc l'absence de contamination est certaine

- si le score différentiel est compris entre v1 et v2, v2 étant par exemple égale à 0.5, l'identité des deux échantillons est possible, et donc la présence de contamination est possible, et

- si le score différentiel est supérieur à v2, il n'y a pas d'identité des deux échantillons et donc la présence de contamination est certaine.

Plus généralement, les différents essais permettent de conclure qu'on peut paramétrer la méthode pour déterminer la présence d'une contamination de l'échantillon si le score différentiel normalisé calculé sur un échantillon incubé pendant un temps t entre 0 et 24H par rapport à un échantillon de référence contaminé par la même bactérie à t=0 ou par rapport à un échantillon de même nature non contaminé dépasse un seuil N1, N1 étant de 0.1 à 10.

**Exemple 6 :** Résultats d'analyse des spectres de masse pour la détection de contamination bactérienne dans des échantillons de concentrés plaquettes contaminés (CP) avant incubation et sans milieu de culture.

1) On a réalisé des identifications bactériennes après inoculation de fortes concentrations de solutions bactériennes, un inoculum de solutions bactériennes de concentration supérieur à $10^7$ ufc/ml de différentes bactéries ayant été ajouté respectivement à différents aux échantillons de CP non contaminés.
Les échantillons contaminés ont subi une méthode de traitement et d'extraction décrite au paragraphe 2) de l'exemple 3 ci-dessus pour analyse spectrale au spectromètre de masse maldi-tof décrit ci-dessus. Les échantillons n'ont pas été incubés à 37°C et ne comportaient pas de milieu de culture.
Les spectres obtenus ont tout d'abord été analysés par le logiciel BIOTYPER®. Cette analyse a eu lieu sans incubation, immédiatement après l'inoculation.
La figure 4 présente des exemples de spectres obtenus à partir d'échantillon non infectés c'est-à-dire avant inoculation (spectres 4a et 4b), et spectres obtenus à partir d'échantillon après inoculation par *Staphylococcus aureus* (spectre 4c), *Escherichia coli* (spectre 4d), *Providencia stuartii* (spectre 4e) et *Pseudomonas aeruginosa* (spectre 4f) et pour de fortes concentrations (supérieures à $10^7$ ufc/mL).
Les pics spécifiques de CP non contaminés n'occultent pas la mise en évidence de pics bactériens. Les flèches montrent la présence de pics supplémentaires dans les spectres 4c à 4f provenant d'échantillons de CP infectés qui correspondent à la présence de protéines bactériennes supplémentaires. La présence de ces pics supplémentaires traduit l'état de contamination. Par ailleurs, on observe comme attendu des pics différents en fonction des espèces.
Les spectres ont été analysés par le logiciel BIOTYPER®. Chaque expérience a été réalisée en quadruple.
Un score d'identification selon le logiciel BIOTYPER® supérieur à 2 est obtenu qui permet une identification du genre et de l'espèce de chaque bactérie par rapport aux spectres de références des dites bactéries de la base de données enregistrée dans BIOTYPER®, c'est-à-dire des spectres de références réalisés sur des bactéries entières et non pas des échantillons de CP infectés et traités selon la présente invention.
Le tableau 2 ci-après présente les valeurs d'indice ou score d'identification S1 obtenu avec le logiciel BIOTYPER® (colonne de droite) pour les spectres obtenus à partir d'échantillons de CP infectés par les différentes bactéries (colonne de gauche) au cours de différents essais.

Tableau 2 :

| Bactérie à $10^7$/ml | | S1 |
|---|---|---|
| *Pseudomonas Aeruginosa* | Essai 1 | 1.967 |
| | Essai 2 | 1.763 |
| | Essai 3 | 2.161 |
| | Essai 4 | 2.134 |
| *Escherichia Coli* | Essai 1 | 2.364 |
| | Essai 2 | 2.420 |
| | Essai 3 | 2.36 |
| | Essai 4 | 2.367 |
| *rovidencia Stuartii* | Essai 1 | 2.186 |
| | Essai 2 | 2.163 |
| | Essai 3 | 2.224 |
| | Essai 4 | 2.154 |

(suite)

| Bactérie à $10^7$/ml | | S1 |
|---|---|---|
| *Staphylococcus A ureus* | Essai 1 | 1.993 |
| | Essai 2 | 2.023 |
| | Essai 3 | 1.237 |
| | Essai 4 | 1.921 |

Les résultats obtenus sont donc globalement satisfaisants pour de très fortes concentrations bactériennes (>$10^7$/ml, sans incubation).

2) Toutefois, comme décrit aux exemples 7 et 8 ci-après, pour des concentrations inférieures et/ou pour certaines bactéries, le score d'identification de BIOTYPER® n'est pas satisfaisant, car l'adjonction des pics spécifiques des composants provenant des plaquettes peut l'induire en erreur de sorte que le logiciel rend alors des résultats faussement négatifs (score trop bas) ou il se trompe dans l'identification proposée voire dans la détection de bactérie.

Ainsi, comme rapporté à l'exemple 7, pour *Klebsiella Pneumoniae* l'identification par BIOTYPER® n'a pas été possible à partir d'extrait protéiques provenant d'échantillon de CP infectés par ladite bactérie quelques soit la concentration en bactérie et ce même après 16h d'incubation.

Il reste que la contamination des CP induit une modification des spectres de sorte que l'observation des spectres est suffisante lorsque l'on ne vise pas l'identification de la bactérie contaminante mais que l'on vise essentiellement la détection d'une contamination par une bactérie quelconque. Dans ce cas, la simple présence de pics additionnels par rapport au spectre caractéristiques de CP non infecté (figure 1, tableau 1) est considérée comme anormal et conduit au retrait de la poche de CP analysée comme pouvant être contaminée. Il convient alors dans un second temps d'identifier si oui ou non il y a vraiment une bactérie mais cette démarche secondaire a un intérêt rétrospectif et épidémiologique, et peut se faire via des techniques classiques: culture sur gélose, analyse de spectre de masse MALDI-TOF classique après isolement de la bactérie.

C'est devant les insuffisances du logiciel BIOTYPER® qu'il a été décidé de développer et d'utiliser une nouvelle méthode de suivi de la pousse bactérienne résultant de l'incubation des échantillons infectés en présence milieu de culture et une nouvelle méthode d'analyse comparative des spectres tel que décrit à l'exemple 9 ci-après

Cette nouvelle méthode d'analyse spectrale ne vise pas ici l'identification mais vise essentiellement la détection d'une contamination par une bactérie par suivi de l'évolution des spectres résultant de la pousse bactérienne dans l'échantillon testé décrit à l'exemple 9 ci-après.

**Exemple 7** : Identification bactérienne après inoculation de concentrations décroissantes de solutions bactériennes et analyse en cinétique du suivi de la pousse bactérienne sans ajout de milieu de culture.

Des solutions de concentrations décroissantes de bactéries ont été inoculées aux CP. L'analyse a été pratiquée sur le logiciel BIOTYPER® selon une cinétique allant de t=0 à t=24h. Les échantillons ont été préparés selon le protocole décrit à l'exemple 3 ci-dessus. Les résultats présentés ici ont été obtenus avec *E. coli, E. faecalis* et *K Pneumoniae.*

Le tableau 3 mentionne les valeurs du score d'identification de BIOTYPER® de spectres obtenus à partir d'extraits protéiques d'échantillons de CP contenant des concentrations initiales croissantes *d'Escherichia Coli* de $10^2$ à $10^7$ ufc/ml sur une période de 24 heures.

Tableau 3 :

| | $10^2$ | $10^3$ | $10^4$ | $10^5$ | $10^6$ | $10^7$ | témoin négatif |
|---|---|---|---|---|---|---|---|
| t0 | 1.2 | 1.2 | 1.2 | 1.2 | 2.0 | 2.2 | 1.2 |
| t=2h | 1.2 | 1.2 | 1.2 | 1.8 | 2.2 | 2.2 | 1.2 |
| t=4h | 1.0 | 1.2 | 2.2 | 2.3 | 2.2 | 2.1 | 1.2 |
| t=6h | 1.9 | 2.3 | 2.3 | 2.3 | 2.2 | 1.9 | 1.2 |
| t=24h | | | | | | | |

Comme le montre le tableau 3, une identification avec un score supérieur à 2 de la bactérie a été obtenue par le logiciel BIOTYPER® en 6 heures suite à une contamination du concentré plaquettaire par 1 ml d'une solution bacté-

rienne de concentration $10^2$ ufc/mL. Dans ces conditions expérimentales, c'est-à-dire en absence d'ajout de milieu de culture, une identification d'espèce est donc obtenue pour E Coli après 6 heures d'incubation pour une concentration de $10^2$ ufc/ml.

Le spectre 5a de la figure 5 montre les pics spécifiques de CP contaminé mais avant incubation et le spectre 5b de la figure 5 correspond à un extrait obtenu à partir d'un échantillon contenant initialement $10^3$/ml de bactérie *E. coli* après incubation 6h. Le spectre 5b illustre que les pics spécifiques de CP (non contaminés) n'occultent pas la mise en évidence de pics bactériens de CP contaminés. Les encadrements de pics montrent les pics bactériens supplémentaires dans le spectre 5b provenant d'échantillons de CP infectés qui correspondent à la présence de protéines bactériennes supplémentaires.

Des expériences similaires ont été réalisées avec d'autres souches bactériennes.

Les résultats obtenus avec *Klebsiella Pneumoniae*, quelques soit la concentration initiale de l'échantillon de $10^2$ à $10^7$ ufc/ml, quelques soit le temps d'incubation de 0 à 16 heures à 37°C, étaient négatifs avec le logiciel BIOTYPER® il n'y a pas de variation significative des spectres représentant une prolifération bactérienne le score d'identification par BIOTYPER® étant toujours inférieur à 1.2. Les scores d'identification obtenus par le logiciel BIOTYPER® ne permettent donc pas d'identifier la présence de la bactérie dans l'échantillon.

Des résultats négatifs similaires ont été obtenus avec d'autres souches bactériennes (résultats non présentés) à des concentrations inférieures à 107 ufc/ml pour les espèces bactériennes de la liste de l'EFS (Etablissement public Français du Sang) mentionnée précédemment.

Au regard de ces résultats, les inventeurs ont décidé d'incuber les préparations de CP infectés dans un milieu de culture selon le protocole décrit à l'exemple 2 et ont obtenu les résultats décrits aux exemples 8 et 9 ci-après.

**Exemple 8 :** Analyse des CP après contamination bactérienne et ajout d'un milieu de culture et suivi de la pousse bactérienne avec le score d'identification du logiciel BIOTYPER®.

Pour favoriser et accélérer la pousse bactérienne et ainsi gagner en sensibilité et en rapidité, l'ajout de milieu de culture a été testé. Les 3 milieux décrits à l'exemple 3 ont été testés. Les échantillons ont été préparés selon le protocole décrit à l'exemple 3 ci-dessus. Les résultats présentés ici ont été obtenus avec *E. coli, E. faecalis* et *K Pneumoniae*.

Le milieu Coeur-Cervelle n'a pas pu s'intégrer dans le protocole. En effet, l'ajout d'acide formique et d'acétonitrile entrainait une gélification de la solution et empêchait ainsi l'obtention de dépôt de qualité sur la cible. Les essais réalisés avec les milieux thiosulfate de sodium et trypticase de soja ont permis d'obtenir des spectres exploitables de qualité.

Des concentrations croissantes de bactéries ont été ajoutées aux CP et incubées selon une cinétique allant de 0 à 16 heures en présence ou non de milieu Thiosulfate de sodium et en présence ou non de milieu trypticase de soja.

Le spectre 6a de la figure 6 montre Les pics spécifiques de CP contaminé mais avant incubation et le spectre 6b de la figure 6 correspond à un extrait obtenu à partir d'un échantillon contenant initialement $10^3$/ml de bactérie *Enterococcus Faecalis* après culture de 6h. Le spectre 6b illustre que les pics spécifiques de CP non contaminé n'occultent pas la mise en évidence de pics bactériens. Les encadrements de pics montrent la présence de pics supplémentaires dans le spectre 6b provenant d'échantillons de CP infectés qui correspondent à la présence de protéines bactériennes supplémentaires.

Les résultats obtenus avec le logiciel BIOTYPER® sont le plus souvent faussement négatifs. Les scores d'identification obtenus avec le logiciel BIOTYPER® avec inoculation de concentrations croissantes de *K Pneumoniae* de $10^2$ à $10^7$ ufc/ml en présence des deux milieux de culture Thiosulfate de Sodium ou trypticase de soja sont inférieurs à 1.2 et ne permettent pas une identification.

Même, après 6 heures de culture pour une concentration initiale à $10^7$ ufc/mL, le logiciel Biotyper® propose une identification incorrecte. En effet, il semble que, dans ce milieu complexe, l'association du spectre plaquettaire au spectre de la bactérie perturbe l'identification de germe par homologie spectrale.

En revanche, les analyses de spectre selon la nouvelle méthode exécutée par le logiciel dénommé logiciel IHU selon la présente invention, permet de détecter une pousse bactérienne en présence d'incubation de l'échantillon infecté avec ou sans milieu de culture comme rapporté à l'exemple 9 ci-après.

**Exemple 9 :** Analyse des CP après contamination bactérienne et ajout d'un milieu de culture et suivi de la pousse bactérienne avec le score d'identification du logiciel IHU.

On a appliqué la méthode d'analyse comparative de spectres décrite à l'exemple 4. A chaque fois les 2 spectres soustraits sont un spectre n°1 à T0=o (avec ajout de milieu et sans ajout de milieu) et un spectre n°2 après incubation de T2= 2h ou T6=6h ou T16=16h (avec ajout de milieu et respectivement sans ajout de milieu)

1) Milieu thiosulfate de sodium

Le tableau 5 ci-après rapporte les valeurs de «score différentiel normalisé » ou « score IHU » obtenues avec le logiciel IHU pour des spectres d'extrait obtenu à partir d'échantillons infectés avec inoculation de concentrations croissantes de K Pneumoniae de $10^2$ à $10^7$ ufc/ml après 0 à 16h d'incubation à 37°C en absence de milieu de culture Thiosulfate de Sodium.

Tableau 5 :

|  | $10^7$ | $10^4$ | $10^3$ | $10^2$ |
|---|---|---|---|---|
| T0 |  |  |  |  |
| T2 | 0,02 | 0,01 | 0,026 | 0,103 |
| T6 | 0,005 | 0,081 | 0,06 | 0,04 |
| T16 | 0,082 | 0,076 | 0,102 | 0,115 |

Le tableau 6 ci-après rapporte les valeurs de score différentiel normalisé ou « score IHU » obtenues avec le logiciel IHU pour des spectres d'extrait obtenu à partir d'échantillons infectés avec inoculation de concentrations croissantes de K Pneumoniae de $10^2$ à $10^7$ ufc/ml après 0 à 16h d'incubation à 37°C en présence de milieu de culture Thiosulfate de Sodium.

Tableau 6 :

|  | $10^7$ | $10^4$ | $10^3$ | $10^2$ |
|---|---|---|---|---|
| T0 |  |  |  |  |
| T2 | 0,013 | 0,037 | 0,021 | 0,033 |
| T6 | 0,095 | 0,055 | 0,057 | 0,067 |
| T16 | 0,148 | 0,164 | 0,14 | 0,121 |

On obtient donc une augmentation du score IHU entre les extrait obtenus à partir d'échantillons ayant subi de 0 et 16 heures d'incubation à 37°C voire seulement 6h, et ce avec et sans milieu thiosulfate de sodium, pour des concentrations initiales de $10^2$ à $10^7$ ufc/mL.

La figure 7 montre que l'augmentation du score 12 (score différentiel normalisé) pour une concentration initiale de $10^7$ ufc/ml bactéries *K.pneumoniae* entre 6h et 16h est plus importante en présence de milieu de culture thiosulfate de sodium (figure 8A) que sans milieu de culture (figure 8B).

Des résultats positifs similaires d'augmentation du score IHU entre les extrait obtenus à partir d'échantillons ayant subi de 0 et 16 heures d'incubation à 37°C voire seulement 6h, et ce avec et sans milieu thiosulfate de sodium, pour des concentrations initiales de $10^2$ à $10^7$ ufc/ml, ont été obtenus pour toutes les bactéries testées , à savoir : *Enterococcus faecalis, Pro videncia stuartii, Klebsiella pneumoniae, Staphylococcus aureus, Staphylococcus epidermidis, Pseudomonas aeruginosa, Klebsiella oxytoca et Enterobacter cloacae.*

Le milieu thiosulfate de sodium permet quelques soit la concentration initiale de la bactérie d'obtenir à T16 une variation significative du score IHU. Cette augmentation du score IHU est le reflet des variations du spectre observées après incubation de l'échantillon en présence de milieu thiosulfate de sodium favorisant la prolifération des bactéries listées.

2) Milieu trypticase de soja

D'autres essais réalisés avec un autre milieu polyvalent de culture bactérienne, le milieu trypticase Soja montrent que le milieu n'est pas un facteur limitant sous réserve que milieu ne contiennent pas de composants gélifiant.

La figure 7A montre les spectres obtenus pour une concentration initiale à $10^2$ufc/ml avant incubation (spectre 7a-1), après 16 heures d'incubation en absence de milieu (spectre 7a-2) ou en présence de milieu trypticase de soja (spectre 7a-3).

La figure 7B montre les spectres obtenus pour une concentration initiale à $10^7$ ufc/ml avant incubation (spectre 7b-1), après 16 heures d'incubation en absence de milieu (spectre 7b-2) ou en présence de milieu trypticase de soja (spectre 7b-3).

Sur les spectres obtenus 7a-3 et 7b-3 des figures 7A et respectivement 7B après 16 heures d'incubation en présence de milieu trypticase de soja on a identifié par des flèches les pics supplémentaires signant la croissance bactérienne détectable.

Le tableau 7A ci-après rapporte les valeurs de score différentiel normalisé ou « score IHU » obtenues avec le logiciel IHU pour des spectres d'extrait obtenu à partir d'échantillons infectés avec inoculation de concentrations croissantes de K Pneumoniae de $10^2$ à $10^7$ ufc/ml après 0 à 16h d'incubation à 37°C en absence de milieu de culture Trypticase de soja.

Tableau 7A :

|     | $10^7$ | $10^4$ | $10^3$ | $10^2$ |
| --- | --- | --- | --- | --- |
| TO  |     |     |     |     |
| T2  | 0,152 | 0,152 | 0,22 | 0,124 |
| T4  | 0,175 | 0,214 | 0,004 | 0,762 |
| T6  | 0,083 | 0,066 | 0,031 | 0,024 |
| T16 | 0,016 | 0,079 | 0,036 | 0,007 |

Les pics p1 et p2 présentent une intensité sensiblement identique entre avant et après incubation et le pic p6 disparait après incubation, ces pics correspondent donc à un composant non bactérien de l'échantillon d'origine. Les pics p3, p4 et p5 présentent une intensité croissante entre avant et après incubation et correspondent donc à un composant d'origine bactérienne.

Le pic P0 est un pic spécifique des plaquettes dans une région dépourvue de pics bactériens. Po diminue lors de la pousse bactérienne et se retrouve en valeur positive après soustraction (figure 3C) du fait d'un effet de compétition en ionisation et/ou dégradation partielle des plaquettes par les bactéries.

Les différents essais permettent de conclure qu'on peut paramétrer la méthode pour déterminer la présence d'une contamination de l'échantillon si le score différentiel normalisé calculé sur un échantillon incubé pendant un temps t entre 0 et 24H par rapport à un échantillon de référence contaminé par la même bactérie à t=0 ou par rapport à un échantillon de même nature non contaminé dépasse un seuil N1, N1 étant de 0.1 à 10.

Le tableau 7B ci-après rapporte les valeurs de score différentiel normalisé ou « score IHU » obtenues avec le logiciel IHU pour des spectres d'extrait obtenu à partir d'échantillons infectés avec inoculation de concentrations croissantes de *K Pneumoniae* de $10^2$ à $10^7$ ufc/ml après 0 à 16h d'incubation à 37°C en présence de milieu de culture trypticase de soja.

Tableau 7B :

|     | $10^7$ | $10^4$ | $10^3$ | $10^2$ |
| --- | --- | --- | --- | --- |
| T0  |     |     |     |     |
| T2  | 0,03 | 0,023 | 0,004 | 0,516 |
| T4  | 0,063 | 0,077 | 0,01 | 0,121 |
| T6  | 0,008 | 0,04 | 0,068 | 1,64 |
| T16 | 0,429 | 0,473 | 0,67 | 1,58 |

L'évolution du score IHU après 0 à 16 heures d'incubation à 37°C avec et sans bouillon trypticase soja permet de détecter une pousse bactérienne pour des concentrations initiales de $10^2$ ufc/ml à $10^7$ ufc/ml.

L'analyse des spectres obtenus avec le logiciel IHU montre une augmentation significative du score IHU reflétant la contamination du CP par la bactérie Klebsiella Pneumoniae.

Les figures 8A et 8B montrent une augmentation importante de l'I2 (score IHU normalisé) entre 6h et 16h pour des concentrations de $10^7$ ufc/ml (figure 8A) et respectivement $10^2$ ufc/ml (figure 8B) de *K.pneumoniae* avec milieu trypticase de soja.

Les figures 9A et 9B représentent des courbes d'évolution du score 12 (score IHU non normalisé) pour *E.coli* à $10^3$ ufc/ml, de 0 à 6h sans milieu de culture (figure 9A) et 0 à 16 h avec milieu trypticase de soja (figure 9B). Dans les deux cas on observe une augmentation significative du score IHU à partir de 4h d'incubation sans milieu de culture et à partir de 6h de culture avec milieu trypticase de culture.

Des résultats positifs similaires d'augmentation du score IHU entre les extrait obtenus à partir d'échantillons ayant subi de 0 et 16 heures d'incubation à 37°C voire seulement 6h, et ce avec et sans milieu trypticase de soja ; pour des concentrations initiales de $10^2$ à $10^7$ UFC/ml, ont été obtenus pour toutes les bactéries testées , à savoir : *Enterococcus faecalis, Pro videncia stuartii, Staphylococcus aureus, Staphylococcus epidermidis, Pseudomonas aeruginosa, Klebsiella oxytoca et Enterobacter cloacae.*

Le milieu trypticase soja a montré un intérêt pour favoriser la prolifération bactérienne dans les CP. En effet, même si l'identification de la bactérie mise en cause n'est pas possible par le logiciel BIOTYPER®, l'ajout du milieu favorise le développement de la bactérie et donc l'apparition de nouveau pics sur le spectre d'analyse. Ces pics supplémentaires signent la contamination du CP.

Le bouillon trypticase soja permet en outre quelques soit la concentration initiale de la bactérie d'obtenir après 16 heures d'incubation à 37°C une variation significative du score IHU. Cette augmentation du score IHU est le reflet des variations du spectre observées après incubation de l'échantillon en présence de trypticase soja favorisant la prolifération de la bactérie.

Les différents essais permettent de conclure qu'on peut paramétrer la méthode pour déterminer la présence d'une contamination de l'échantillon de concentré de plaquettes si le ratio St/So entre :

- le score différentiel normalisé (St), calculé sur un échantillon incubé pendant un temps t entre 0 et 24H par rapport à un échantillon de référence de concentré de plaquettes contaminé par la même bactérie à t=0 ou par rapport à un échantillon de de concentré de plaquettes non contaminé, et

- le score différentiel calculé à t=0 (So) par rapport à un échantillon de même nature non contaminé dépasse un seuil N2, N2 étant de préférence d'au moins 1.5.

## Revendications

1. Méthode de détection de la contamination par un microbe d'un échantillon de produit sanguin contenant des protéines et/ou macromolécules et/ou des cellules et/ou fragments de cellules du sang ou destinés à complémenter le sang, dans lequel un dit microbe peut proliférer, ledit microbe étant de préférence une bactérie ou une levure, comprenant les étapes suivantes dans lesquelles :

   a) on réalise un traitement de lyse d'au moins le ou les dits microbes pouvant être contenus dans ledit échantillon de dit produit sanguin à analyser après un temps t d'incubation,
   b) on réalise un spectre par spectrométrie de masse de préférence du type MALDI-TOF dudit échantillon de dit produit sanguin ou d'un extrait protéique dudit échantillon de dit produit sanguin ainsi lysé à un temps t, et
   c) on détermine si ledit échantillon à analyser est contaminé par comparaison du spectre obtenu avec au moins un spectre de spectrométrie de masse du type MALDI-TOF choisi parmi les spectres suivants:

      c.1) au moins un spectre de référence choisi parmi :

         c.1.1- au moins un spectre d'un dit échantillon standard ou respectivement d'un dit extrait protéique dudit échantillon standard, ledit échantillon standard ou respectivement ledit extrait de dit échantillon standard étant un échantillon de même nature que ledit échantillon à analyser, mais non contaminé, et
         c.1.2- des spectres de dits échantillons standards ou d'extraits protéiques d'échantillons standards de même nature contaminés chacun par respectivement un microbe connu différent, les dits échantillons standards ou dits extraits ayant subis le même traitement de lyse, et

      c.2) au moins un spectre d'un dit échantillon d'origine ou dit extrait dudit échantillon d'origine ayant subi le même traitement de lyse avant incubation ou après un temps t' d'incubation différent de t.

2. Méthode de détection de la contamination par un microbe, de préférence une bactérie, d'un échantillon de dit produit sanguin à analyser selon la revendication 1 par suivi de la croissance dudit microbe comprenant les étapes successives suivantes dans lesquelles :

   a.1) on réalise une culture dudit microbe en ajoutant un milieu de culture dudit microbe dans dudit échantillon, pendant un temps t de préférence compris entre 6 et 24 heures, et
   a.2) on réalise une lyse cellulaire des microbes et autres cellules dudit échantillon, puis
   a.3) on réalise une extraction des protéines à partir d'un lysat dudit échantillon après culture, de l'étape a.2), et

b) on réalise un spectre par spectrométrie de masse du type MALDI-TOF dudit extrait protéique obtenu à l'étape a.3), et

c) on détermine si ledit échantillon est contaminé par comparaison du spectre obtenu à l'étape b) avec au moins un spectre de spectrométrie de masse du type MALDI-TOF choisi parmi les spectres suivants:

    c.1) au moins un spectre de référence choisi parmi :

        - c.1.1) un spectre d'un extrait protéique de référence d'un échantillon standard de même nature mais non contaminé, ayant subi le même traitement de lyse et extraction des étapes a.2) et a.3), et
        - c.1.2) des spectres de référence d'extraits protéiques d'échantillons standards de même nature contaminés par des microbes connus, ayant subis le même traitement au moins des étapes a.1) et a.2), de préférence ayant subis le même traitement au moins des a.1 à a.3), et

    c.2) au moins un spectre d'un extrait protéique obtenu par extraction des protéines à partir d'un traitement de lyse et extraction dudit échantillon d'origine selon les étapes a.2) et a.3) avant ou après culture de l'étape a.1) pendant un temps t' inférieur à t.

**3.** Méthode selon la revendication 1 ou 2, **caractérisée en ce que** ledit échantillon de produit sanguin à analyser est constitué d'un milieu liquide complexe contenant des cellules ou fragments de cellules eucaryotes, de préférences des cellules ou fragments de cellules du sang ou des cellules de la moelle osseuse.

**4.** Méthode selon l'une des revendications 1 à 3, **caractérisée en ce que** ledit échantillon de produit sanguin est un échantillon humain ou animal contenant tout ou partie du sang choisi parmi le sang total, un concentré de globules rouges, un concentré de plaquettes ou du plasma frais.

**5.** Méthode selon la revendication 4, **caractérisée en ce que** ledit échantillon est un concentré de plaquettes et à l'étape c), on réalise l'étape c.1) en comparant le spectre de l'extrait protéique de lysat de concentré de plaquettes à analyser avec un spectre de référence qui est un spectre d'un extrait protéique de concentré plaquettaire standard non contaminé et on détecte une contamination si on retrouve dans le spectre de l'échantillon analysé au moins les pics spécifiques de plus grandes intensités du spectre de référence.

**6.** Méthode selon l'une des revendications 1 à 5, **caractérisée en ce qu'**à l'étape a), ledit extrait protéique est obtenu en effectuant les étapes successives suivantes :

    1) on élimine le liquide du dit échantillon à analyser, et on récupère un premier résidu solide, et
    2) de préférence, on ajoute une solution de détergent au dit premier résidu solide, et
    3) on élimine le liquide à nouveau et on récupère un deuxième résidu solide, et
    4) on réalise une lyse des cellules présente dans ledit deuxième résidu solide, de préférence une lyse chimique, et
    5) on solubilise les protéines présentes dans ledit deuxième résidu solide, de préférence avec une solution de solvant, et
    6) on élimine les substances non solubilisées, pour récupérer une solution essentiellement constituée de protéines entières.

**7.** Méthode selon la revendication 6, **caractérisée en ce que** pour traiter un échantillon de concentré de plaquettes, ledit extrait protéique est réalisé en effectuant au moins les étapes successives suivantes :

    1) on élimine le liquide du dit échantillon à analyser, en réalisant une première centrifugation et éliminant le surnageant de centrifugation, et en récupérant un premier résidu solide qui est le culot de première centrifugation, et
    2) on ajoute une solution de détergent au dit premier résidu solide, de préférence une solution de saponine, et
    3) on récupère un deuxième résidu solide par une deuxième centrifugation et en éliminant le surnageant et en récupérant un deuxième résidu solide qui est le culot de deuxième centrifugation, et
    4) on réalise une lyse chimique des cellules présente dans ledit deuxième résidu solide, de préférence on réalise un traitement chimique avec une solution acide d'acide formique, et
    5) on solubilise les protéines présentes dans ledit deuxième résidu solide de lysat cellulaire, de préférence avec une solution d'acide formique et acétonitrile, et
    6) on élimine les substances non solubilisées, par une troisième centrifugation et en éliminant le culot et récupérant le surnageant de troisième centrifugation, pour récupérer une solution essentiellement constituée de protéines entières.

8. Méthode selon l'une des revendications 4 à 7, **caractérisée en ce que** ledit spectre de référence spécifique de dit extrait de concentré de plaquettes sanguines non contaminé comprend au moins les pics caractéristiques de plus grande intensités, dans le tableau 1 suivant dans lequel les différents pics caractéristiques sont **caractérisés par** leurs coordonnées en abscisse m/z (Da) et leurs coordonnées en ordonnée d'intensité relative exprimées en S/N (intensité du pic/intensité du bruit de fond) les valeurs des masses moléculaires m/z pouvant varier jusqu'à $\pm 1$ Da de la valeur moyenne mentionnée dans le tableau 1, et les valeurs d'intensité relative pouvant varier jusqu'à $\pm 20\%$ de la valeur moyenne d'intensité relative mentionnée :

Tableau 1 :

| Index | m/z | Intensité (S/N) | Index | m/z | Intensité (S/N) |
|---|---|---|---|---|---|
| 2 | 2039,33 | 0,82 | 45 | 5941,47 | 12,96 |
| 3 | 2053,09 | 0,96 | 46 | 6123,11 | 4,61 |
| 4 | 2172,47 | 2,81 | 47 | 6195,2 | 4,99 |
| 5 | 2188,26 | 2,08 | 48 | 6253,84 | 12,31 |
| 6 | 2206,14 | 1,49 | 49 | 6329,64 | 34,15 |
| 7 | 2335,49 | 0,81 | 50 | 6399,07 | 4,69 |
| 8 | 2587,08 | 9,77 | 51 | 6437,89 | 19,1 |
| 9 | 2677,66 | 6,53 | 52 | 6636,41 | 10,92 |
| 10 | 2824,37 | 44,11 | 53 | 6667,28 | 30,17 |
| 11 | 3124,2 | 2,88 | 54 | 6725,81 | 41,44 |
| 12 | 3170,04 | 2,43 | 55 | 7193,2 | 13,26 |
| 13 | 3332,51 | 4,02 | 56 | 7307,12 | 1,57 |
| 14 | 3372,52 | 27,37 | 57 | 7355,33 | 2,03 |
| 15 | 3443,5 | 48,02 | 58 | 7437,27 | 19,85 |
| 16 | 3487,23 | 15,79 | 59 | 7567,73 | 42,56 |
| 17 | 3717,3 | 2,85 | 60 | 7642,27 | 28,22 |
| 18 | 3783,47 | 6,75 | 61 | 7768,13 | 745,58 |
| 19 | 3820,02 | 5,97 | 62 | 7839,16 | 152,91 |
| 20 | 3883,82 | 343,89 | 63 | 7924,93 | 114,32 |
| 21 | 3920,17 | 34,15 | 64 | 8022,69 | 21,31 |
| 22 | 3963,03 | 32,32 | 65 | 8144,94 | 177,92 |
| 23 | 4049,38 | 1,6 | 66 | 8211,76 | 12,77 |
| 24 | 4072,05 | 48,19 | 67 | 8584,21 | 37,68 |
| 25 | 4292,54 | 15,29 | 68 | 8652,46 | 28,04 |
| 26 | 4326,29 | 5,36 | 69 | 8719,83 | 16,44 |
| 27 | 4481,88 | 47,61 | 70 | 8963,52 | 106,86 |
| 28 | 4568,92 | 4,21 | 71 | 9039,83 | 6,86 |
| 29 | 4623,37 | 14,23 | 72 | 9136,49 | 7,45 |
| 30 | 4646,43 | 14,15 | 73 | 9249,43 | 26,67 |
| 31 | 4688,05 | 31,78 | 74 | 9291,19 | 35,04 |

(suite)

| Index | m/z | Intensité (S/N) | Index | m/z | Intensité (S/N) |
|---|---|---|---|---|---|
| 32 | 4732,37 | 17,33 | 75 | 9375,12 | 58,79 |
| 33 | 4760,03 | 62,14 | 76 | 9464,76 | 35,82 |
| 34 | 4813,43 | 38,33 | 77 | 9519,48 | 89,66 |
| 35 | 4896,95 | 40,58 | 78 | 9621,13 | 71,71 |
| 36 | 4966,02 | 17,88 | 79 | 10102,91 | 69,65 |
| 37 | 5052,1 | 44,86 | 80 | 10189,05 | -0,33 |
| 38 | 5137,16 | 4,66 | 81 | 10262,85 | 2,29 |
| 39 | 5268,85 | 46,78 | 82 | 10617,08 | 29,7 |
| 40 | 5310,04 | 31,23 | 83 | 10701,45 | 1,97 |
| 41 | 5360,04 | 138,98 | 84 | 10843,44 | 40,58 |
| 42 | 5422,73 | 19,14 | 85 | 12392,91 | 2,15 |
| 43 | 5652,14 | 229,61 | 86 | 12503,41 | 9,72 |
| 44 | 5698,21 | 13,9 | 87 | 13441.97 | 3.28 |

**9.** Méthode selon l'une des revendications 1 à 8, **caractérisé en ce qu'**à l'étape c), on réalise la comparaison de 2 spectres essentiellement en calculant la soustraction entre les aires totales respectives des 2 spectres, de préférence 2 spectres d'extraits protéiques du même échantillon d'origine après 2 temps d'incubation ou culture différents.

**10.** Méthode selon la revendication 9, **caractérisée en ce qu'**à l'étape c), on réalise au moins les étapes successives suivantes :

i.1) on relève les intensités yi de respectivement chacun des points d'abscisse xi correspondant aux valeurs de rapport massique m/z de chaque spectre, et
i.2) pour chaque valeur de xi on calcule une intensité résultante yi3 par soustraction des intensités yi1 et yi2 respectives dudit spectre à analyser (yi1) et respectivement dudit spectre de référence (yi2) :yi3=yi2-yi1, et
i.3) on calcule la somme des intensités résultantes yi3 pour toutes les valeurs xi pour obtenir un score de caractérisation dénommé score différentiel $\sum_{i=0}^{i=n} |yi3|$.

**11.** Méthode selon la revendication 10, **caractérisée en ce qu'**à l'étape c), on réalise au moins les étapes suivantes :

i.1) on normalise les intensités yi de respectivement chacun des points d'abscisse xi correspondant aux valeurs de rapport massique m/z de chaque spectre en divisant les intensités yi de chaque point d'abscisse xi (m/z) par l'intensité du pic de plus grande intensité, et
i.2) pour chaque valeur de xi on calcule une intensité résultante normalisée yi3 par soustraction des intensités normalisées respectives de deux spectres yi1 et yi2 :yi3=yi2-yi1, et
i.3) on calcule la somme des intensités résultantes normalisées yi3 pour toutes les valeurs xi pour obtenir un score de caractérisation dénommé score différentiel = $\sum_{i=0}^{i=n} |yi3|$, et de préférence on la normalise en la divisant par la somme des intensités pour toutes les valeurs xi de l'un des spectres, de préférence le spectre de plus petite somme des intensités pour toutes les valeurs xi, pour obtenir un score différentiel normalisé =

$$\sum_{i=0}^{i=n} |yi3| \Big/ \sum_{i=0}^{i=n} yip \text{ avec p=1 ou 2.}$$

**12.** Méthode selon la revendication 11, **caractérisée en ce qu'**à l'étape i.3), on réalise les étapes i.3a) et i.3b) de filtrage préalables avant l'étape 3c) suivantes :

i.3a) pour chaque valeur de xi, on enlève le bruit de fond en donnant la valeur d'intensité résultante normalisée yi3=0 si yi3 est inférieur à un seuil $Sy_1$, $Sy_1$ étant inférieur à 10% de la valeur $yi_{max}$ du pic de plus grande

intensité, de préférence $Sy_1$ = 5 % de la valeur $yi_{max}$ du pic de plus grande intensité, et

i.3b) on réalise un filtrage en donnant la valeur yi3=0 pour les valeurs de xi pour lesquels à xi' avec xi'-xi inférieur à un seuil Sx, de préférence Sx étant inférieur ou égal à 10Da, l'intensité résultante normalisée yi3' est de signe opposée à yi3 et de valeur absolue d'au moins une valeur seuil relative de $Sy_2$% de celle de yi3, $Sy_2$ étant de préférence d'au moins 70% de celle de yi3, et

i.3c) on calcule la somme des intensités restantes après filtrage yi3 pour toutes les valeurs xi pour obtenir un score de caractérisation dénommé score différentiel = $\sum_{i=0}^{i=n} |yi3|$, et de préférence on la normalise en la divisant par la somme des intensités pour toutes les valeurs xi de l'un des spectres, de préférence le spectre de plus petite somme des intensités pour toutes les valeurs xi, pour obtenir un score différentiel normalisé =

$$\sum_{i=0}^{i=n} |yi3| / \sum_{i=0}^{i=n} yip \quad \text{avec p = 1 ou 2.}$$

**13.** Méthode selon l'une des revendications 1 à 12, **caractérisée en ce qu'**on cherche à détecter la présence d'une bactérie choisie au moins parmi les bactéries suivantes :-*Escherichia coli, Enterococcus faecalis, Providencia stuartii, Klebsiella pneumoniae, Staphylococcus aureus, Staphylococcus epidermidis, Pseudomonas aeruginosa, Klebsiella oxytoca et Enterobacter cloacae.*

**14.** Méthode selon l'une des revendications 2 à 13, **caractérisée en ce qu'**à l'étape b) on réalise une culture avec un milieu de culture polyvalent de bactérie.

**15.** Méthode selon l'une des revendications 4 à 14, **caractérisée en ce qu'**on détermine si l'échantillon dit produit sanguin à analyser, de préférence un concentré de plaquettes est infecté par une bactérie, de préférence à une concentration d'au moins 10 bactéries/ml, si ledit score différentiel calculé à partir des 2 spectres varie, de préférence augmente, entre:

- une première valeur de score de comparaison entre un premier spectre d'un dit extrait protéique dudit échantillon testé avant culture ou après culture au temps t0 et un deuxième spectre d'un dit extrait protéique dudit échantillon testé respectivement après culture à t1, t1 étant supérieur à t0, de préférence to étant de 0 à 6h et t1 étant de 2 à 24h, de préférence encore to étant de 0 à 2h et t1 étant de 2 à 6h, et

- au moins une seconde valeur de score de comparaison entre un premier spectre d'un dit extrait protéique dudit échantillon testé avant culture ou après culture au temps t0 et un deuxième spectre d'un dit extrait protéique dudit échantillon testé respectivement après culture à t2, t2 étant supérieur à t1, de préférence to étant de 0 à 2h , t1 étant de 2 à 6h et t2 étant de 4 à 16h.

## Patentansprüche

**1.** Verfahren zur Erkennung der Kontamination einer Probe eines Blutproduktes, das Proteine und/oder Makromoleküle und/oder Zellen und/oder Fragmente von Zellen des Blutes oder zur Ergänzung des Blutes enthält, durch eine Mikrobe, wobei eine Mikrobe proliferieren kann, wobei die Mikrobe vorzugsweise eine Bakterie oder eine Hefe ist, umfassend die folgenden Schritte, bei denen:

a) eine Lysebehandlung wenigstens der Mikrobe oder Mikroben, die in der zu analysierenden Probe des Blutproduktes enthalten sein können, nach einer Inkubationszeit t durchgeführt wird,

b) ein Spektrum durch Massenspektrometrie vorzugsweise vom Typ MALDI-TOF der Probe des Blutproduktes oder eines Proteinextraktes der Probe des Blutproduktes, die bzw. der auf diese Weise lysiert wurde, zu einem Zeitpunkt t erstellt wird, und

c) bestimmt wird, ob die zu analysierende Probe kontaminiert ist, indem das erhaltene Spektrum mit wenigstens einem Spektrum einer Massenspektrometrie vom Typ MALDI-TOF, das aus den nachfolgenden Spektren ausgewählt ist, verglichen wird:

c.1) wenigstens einem Referenzspektrum, das ausgewählt ist aus:

c.1.1- wenigstens einem Spektrum einer Standardprobe bzw. eines Proteinextraktes der Standardprobe, wobei die Standardprobe bzw. der Extrakt der Standardprobe eine Probe gleicher Art wie die zu analysierende Probe, jedoch nicht kontaminiert, ist, und

c.1.2- Spektren von Standardproben oder von Proteinextrakten von Standardproben gleicher Art, die jeweils durch je eine verschiedene bekannte Mikrobe kontaminiert sind, wobei die Standardproben oder Extrakte der gleichen Lysebehandlung unterzogen worden sind, und

c.2) wenigstens einem Spektrum einer Ursprungsprobe oder eines Extraktes der Ursprungsprobe, die bzw. der der gleichen Lysebehandlung vor Inkubation oder nach einer von t verschiedenen Inkubationszeit t' unterzogen worden ist.

2. Verfahren zur Erkennung der Kontamination einer zu analysierenden Probe eines Blutproduktes durch eine Mikrobe, vorzugsweise eine Bakterie, nach Anspruch 1, durch Verfolgen des Wachstums der Mikrobe, umfassend die folgenden aufeinanderfolgenden Schritte, bei denen:

a.1) für eine Zeitdauer t, die vorzugsweise zwischen 6 und 24 Stunden beträgt, durch Zugabe eines Mediums zum Kultivieren der Mikrobe zu der Probe eine Kultivierung durchgeführt wird, und
a.2) eine Zelllyse der Mikroben und weiterer Zellen der Probe durchgeführt wird, anschließend
a.3) eine Extraktion der Proteine aus einem Lysat der Probe nach Kultivierung aus Schritt a.2) durchgeführt wird, und
b) ein Spektrum durch Massenspektrometrie vom Typ MALDI-TOF des bei Schritt a.3) erhaltenen Proteinextraktes erstellt wird, und
c) bestimmt wird, ob die Probe kontaminiert ist, indem das bei Schritt b) erhaltene Spektrum mit wenigstens einem Spektrum einer Massenspektrometrie vom Typ MALDI-TOF, das aus den nachfolgenden Spektren ausgewählt ist, verglichen wird:

c.1) wenigstens einem Referenzspektrum, das ausgewählt ist aus:

- c.1.1) einem Spektrum eines Referenzproteinextraktes einer Standardprobe gleicher Art, jedoch nicht kontaminiert, welcher der gleichen Lysebehandlung und Extraktion der Schritte a.2) und a.3) unterzogen worden ist, und
- c.1.2) Referenzspektren von Proteinextrakten von Standardproben gleicher Art, die durch bekannte Mikroben kontaminiert sind, die die gleiche Behandlung wenigstens der Schritte a.1) und a.2) unterzogen worden sind, die vorzugsweise der gleichen Behandlung wenigstens von a.1 bis a.3) unterzogen worden sind, und

c.2) wenigstens einem Spektrum eines Proteinextraktes, der durch Extraktion der Proteine mit Hilfe einer Lysebehandlung und Extraktion der Ursprungsprobe gemäß der Schritte a.2) und a.3) vor oder nach Kultivierung des Schrittes a.1) über einen Zeitraum t' kleiner als t erhalten wird.

3. Verfahren nach Anspruch 1 oder 2, **dadurch gekennzeichnet, dass** die zu analysierende Probe des Blutproduktes von einem komplexen flüssigen Medium, das Zellen oder Fragmente von eukaryotischen Zellen, vorzugsweise Zellen oder Fragmente von Zellen des Blutes oder Zellen des Knochenmarks enthält, gebildet ist.

4. Verfahren nach einem der Ansprüche 1 bis 3, **dadurch gekennzeichnet, dass** die Probe des Blutproduktes eine menschliche oder tierische Probe ist, die das Blut ausgewählt aus Vollblut, einem Konzentrat aus roten Blutkörperchen, einem Konzentrat aus Blutplättchen oder frischem Plasma in Gänze oder teilweise enthält.

5. Verfahren nach Anspruch 4, **dadurch gekennzeichnet, dass** die Probe ein Konzentrat aus Blutplättchen ist und bei Schritt c) der Schritt c.1) dadurch vollzogen wird, dass das Spektrum des Proteinextraktes von Lysat von zu analysierendem Blutplättchen-Konzentrat mit einem Referenzspektrum verglichen wird, das ein Spektrum eines Proteinextraktes von nicht kontaminiertem Standard-Blutplättchen-Konzentrat ist, und eine Kontamination erkannt wird, wenn in dem Spektrum der analysierten Probe wenigstens die spezifischen Peaks mit höheren Intensitäten des Referenzspektrums vorgefunden werden.

6. Verfahren nach einem der Ansprüche 1 bis 5, **dadurch gekennzeichnet, dass** bei Schritt a) der Proteinextrakt dadurch erhalten wird, dass die folgenden aufeinanderfolgenden Schritte durchgeführt werden:

1) die Flüssigkeit wird aus der zu analysierenden Probe entfernt, und es wird ein erster fester Rückstand gewonnen, und
2) vorzugsweise wird dem ersten festen Rückstand eine Detergenslösung zugegeben, und
3) die Flüssigkeit wird erneut entfernt, und es wird ein zweiter fester Rückstand gewonnen, und

4) es wird eine Lyse der in dem zweiten festen Rückstand vorhandenen Zellen, vorzugsweise eine chemische Lyse durchgeführt, und

5) die Proteine, die in dem zweiten festen Rückstand vorhanden sind, werden solubilisiert, vorzugsweise mit einer Lösungsmittellösung, und

6) die nicht solubilisierten Substanzen werden entfernt, um eine im Wesentlichen von vollständigen Proteinen gebildete Lösung zu gewinnen.

7. Verfahren nach Anspruch 6, **dadurch gekennzeichnet, dass** zur Behandlung einer Probe von Blutplättchen-Konzentrat der Proteinextrakt dadurch hergestellt wird, dass wenigstens die folgenden aufeinanderfolgenden Schritte durchgeführt werden:

1) die Flüssigkeit wird aus der zu analysierenden Probe entfernt, indem eine erste Zentrifugation durchgeführt und der Zentrifugierüberstand entfernt wird und indem ein erster fester Rückstand, welcher der Rückstand der ersten Zentrifugation ist, gewonnen wird, und

2) dem ersten festen Rückstand wird eine Detergenslösung, vorzugsweise eine Saponin-Lösung zugegeben, und

3) ein zweiter fester Rückstand wird durch eine zweite Zentrifugation und durch Entfernen des Überstandes und Gewinnen eines zweiten festen Rückstandes, welcher der Rückstand der zweiten Zentrifugation ist, gewonnen, und

4) es wird eine chemische Lyse der in dem zweiten festen Rückstand vorhandenen Zellen durchgeführt, vorzugsweise wird eine chemische Behandlung mit einer sauren Lösung von Ameisensäure durchgeführt, und

5) die Proteine, die in dem zweiten festen Rückstand von Zelllysat vorhanden sind, werden solubilisiert, vorzugsweise mit einer Lösung von Ameisensäure und Acetonitril, und

6) die nicht solubilisierten Substanzen werden durch eine dritte Zentrifugation und durch Entfernen des Rückstandes und Gewinnen des Überstandes der dritten Zentrifugation entfernt, um eine im Wesentlichen von vollständigen Proteinen gebildete Lösung zu gewinnen.

8. Verfahren nach einem der Ansprüche 4 bis 7, **dadurch gekennzeichnet, dass** das Referenzspektrum, das für einen Extrakt von nicht kontaminiertem Blutplättchen-Konzentrat spezifisch ist, wenigstens die charakteristischen Peaks mit höheren Intensitäten umfasst, wobei in der folgenden Tabelle 1, in der die verschiedenen charakteristischen Peaks durch ihre Koordinaten auf der Abszisse m/z (Da) und ihre Koordinaten der relativen Intensität auf der Ordinate, ausgedrückt in S/N (Intensität des Peaks/Intensität des Grundrauschens), gekennzeichnet sind, die Werte der molaren Massen m/z bis zu $\pm 1$ Da des in der Tabelle 1 erwähnten Mittelwertes variieren können und die Werte der relativen Intensität bis zu $\pm 20$ % des erwähnten Mittelwertes der relativen Intensität variieren können:

Tabelle 1:

| Index | m/z | Intensität (S/N) | Index | m/z | Intensität (S/N) |
|---|---|---|---|---|---|
| 2 | 2039,33 | 0,82 | 45 | 5941,47 | 12,96 |
| 3 | 2053,09 | 0,96 | 46 | 6123,11 | 4,61 |
| 4 | 2172,47 | 2,81 | 47 | 6195,2 | 4,99 |
| 5 | 2188,26 | 2,08 | 48 | 6253,84 | 12,31 |
| 6 | 2206,14 | 1,49 | 49 | 6329,64 | 34,15 |
| 7 | 2335,49 | 0,81 | 50 | 6399,07 | 4,69 |
| 8 | 2587,08 | 9,77 | 51 | 6437,89 | 19,1 |
| 9 | 2677,66 | 6,53 | 52 | 6636,41 | 10,92 |
| 10 | 2824,37 | 44,11 | 53 | 6667,28 | 30,17 |
| 11 | 3124,2 | 2,88 | 54 | 6725,81 | 41,44 |
| 12 | 3170,04 | 2,43 | 55 | 7193,2 | 13,26 |
| 13 | 3332,51 | 4,02 | 56 | 7307,12 | 1,57 |
| 14 | 3372,52 | 27,37 | 57 | 7355,33 | 2,03 |

(fortgesetzt)

| Index | m/z | Intensität (S/N) | Index | m/z | Intensität (S/N) |
|---|---|---|---|---|---|
| 15 | 3443,5 | 48,02 | 58 | 7437,27 | 19,85 |
| 16 | 3487,23 | 15,79 | 59 | 7567,73 | 42,56 |
| 17 | 3717,3 | 2,85 | 60 | 7642,27 | 28,22 |
| 18 | 3783,47 | 6,75 | 61 | 7768,13 | 745,58 |
| 19 | 3820,02 | 5,97 | 62 | 7839,16 | 152,91 |
| 20 | 3883,82 | 343,89 | 63 | 7924,93 | 114,32 |
| 21 | 3920,17 | 34,15 | 64 | 8022,69 | 21,31 |
| 22 | 3963,03 | 32,32 | 65 | 8144,94 | 177,92 |
| 23 | 4049,38 | 1,6 | 66 | 8211,76 | 12,77 |
| 24 | 4072,05 | 48,19 | 67 | 8584,21 | 37,68 |
| 25 | 4292,54 | 15,29 | 68 | 8652,46 | 28,04 |
| 26 | 4326,29 | 5,36 | 69 | 8719,83 | 16,44 |
| 27 | 4481,88 | 47,61 | 70 | 8963,52 | 106,86 |
| 28 | 4568,92 | 4,21 | 71 | 9039,83 | 6,86 |
| 29 | 4623,37 | 14,23 | 72 | 9136,49 | 7,45 |
| 30 | 4646,43 | 14,15 | 73 | 9249,43 | 26,67 |
| 31 | 4688,05 | 31,78 | 74 | 9291,19 | 35,04 |
| 32 | 4732,37 | 17,33 | 75 | 9375,12 | 58,79 |
| 33 | 4760,03 | 62,14 | 76 | 9464,76 | 35,82 |
| 34 | 4813,43 | 38,33 | 77 | 9519,48 | 89,66 |
| 35 | 4896,95 | 40,58 | 78 | 9621,13 | 71,71 |
| 36 | 4966,02 | 17,88 | 79 | 10102,91 | 69,65 |
| 37 | 5052,1 | 44,86 | 80 | 10189,05 | -0,33 |
| 38 | 5137,16 | 4,66 | 81 | 10262,85 | 2,29 |
| 39 | 5268,85 | 46,78 | 82 | 10617,08 | 29,7 |
| 40 | 5310,04 | 31,23 | 83 | 10701,45 | 1,97 |
| 41 | 5360,04 | 138,98 | 84 | 10843,44 | 40,58 |
| 42 | 5422,73 | 19,14 | 85 | 12392,91 | 2,15 |
| 43 | 5652,14 | 229,61 | 86 | 12503,41 | 9,72 |
| 44 | 5698,21 | 13,9 | 87 | 13441.97 | 3.28 |

9. Verfahren nach einem der Ansprüche 1 bis 8, **dadurch gekennzeichnet, dass** bei Schritt c) der Vergleich von 2 Spektren im Wesentlichen durch Berechnen der Subtraktion zwischen den jeweiligen Gesamtflächen der 2 Spektren, vorzugsweise 2 Spektren von Proteinextrakten der gleichen Ursprungsprobe nach 2 unterschiedlichen Inkubations- oder Kultivierungszeiten durchgeführt wird.

10. Verfahren nach Anspruch 9, **dadurch gekennzeichnet, dass** bei Schritt c) wenigstens die folgenden aufeinanderfolgenden Schritte durchgeführt werden:

i.1) es werden die Intensitäten $y_i$ jeweils eines jeden der Abszissen-Punkte $x_i$, die den Masseverhältniswerten m/z eines jeden Spektrums entsprechen, aufgenommen, und

i.2) für einen jeden Wert von xi wird eine resultierende Intensität yi3 durch Subtraktion der jeweiligen Intensitäten yi1 und yi2 des zu analysierenden Spektrums (yi1) bzw. des Referenzspektrums (yi2) : yi3 = yi2 - yi1 berechnet, und

i.3) es wird die Summe der resultierenden Intensitäten yi3 für alle Werte xi berechnet, um einen als Differenzscore $= \sum_{i=0}^{i=n} |yi3|$ bezeichneten Charakterisierungsscore zu erhalten.

**11.** Verfahren nach Anspruch 10, **dadurch gekennzeichnet, dass** bei Schritt c) wenigstens die folgenden Schritte durchgeführt werden:

i.1) die Intensitäten yi jeweils eines jeden der Abszissen-Punkte xi, die den Masseverhältniswerten m/z eines jeden Spektrums entsprechen, werden normalisiert, indem die Intensitäten yi eines jeden Abszissen-Punktes xi (m/z) durch die Intensität des Peaks mit höherer Intensität dividiert werden, und

i.2) für einen jeden Wert von xi wird eine normalisierte resultierende Intensität yi3 durch Subtraktion der jeweiligen normalisierten Intensitäten von zwei Spektren yi1 und yi2 : yi3 = yi2 - yi1 berechnet, und

i.3) es wird die Summe der normalisierten resultierenden Intensitäten yi3 für alle Werte xi berechnet, um einen als Differenzscore $= \sum_{i=0}^{i=n} |yi3|$ bezeichneten Charakterisierungsscore zu erhalten, und vorzugsweise wird sie normalisiert, indem sie durch die Summe der Intensitäten für alle Werte xi von einem der Spektren dividiert wird, vorzugsweise dem Spektrum mit kleinerer Summe der Intensitäten für alle Werte xi, um einen normalisierten Differenzscore $= \sum_{i=0}^{i=n} |yi3| / \sum_{i=0}^{i=n} yip$, mit p = 1 oder 2, zu erhalten.

**12.** Verfahren nach Anspruch 11, **dadurch gekennzeichnet, dass** bei Schritt i.3) die folgenden vorhergehenden Filterschritte i.3a) und i.3b) vor Schritt 3c) durchgeführt werden:

i.3a) für jeden Wert von xi wird das Grundrauschen entfernt durch Vorgeben des Wertes der normalisierten resultierenden Intensität yi3 = 0, wenn yi3 kleiner als ein Schwellwert $Sy_1$ ist, wobei $Sy_1$ kleiner als 10 % des Wertes $yi_{max}$ des Peaks mit höherer Intensität, vorzugsweise $Sy_1$ = 5 % des Wertes $yi_{max}$ des Peaks mit höherer Intensität ist, und

i.3b) es wird ein Filtern durchgeführt durch Vorgeben des Wertes yi3 = 0 für die Werte von xi, bei denen bei xi', mit xi'-xi kleiner als ein Schwellwert Sx, wobei vorzugsweise Sx kleiner oder gleich 10Da, die normalisierte resultierende Intensität yi3' ein zu yi3 entgegengesetztes Vorzeichen und einen Absolutwert von wenigstens einem relativen Schwellwert $Sy_2$% von demjenigen von yi3 aufweist, wobei $Sy_2$ vorzugsweise wenigstens 70 % von dem von yi3 beträgt, und

i.3c) es wird die Summe der nach Filtern verbleibenden Intensitäten yi3 für alle Werte xi berechnet, um einen als Differenzscore $= \sum_{i=0}^{i=n} |yi3|$ bezeichneten Charakterisierungsscore zu erhalten, und vorzugsweise wird sie normalisiert, indem sie durch die Summe der Intensitäten für alle Werte xi von einem der Spektren dividiert wird, vorzugsweise dem Spektrum mit kleinerer Summe der Intensitäten für alle Werte xi, um einen normalisierten Differenzscore $= \sum_{i=0}^{i=n} |yi3| / \sum_{i=0}^{i=n} yip$, mit p = 1 oder 2, zu erhalten.

**13.** Verfahren nach einem der Ansprüche 1 bis 12, **dadurch gekennzeichnet, dass** versucht wird, das Vorliegen einer Bakterie zu erkennen, die wenigstens aus den folgenden Bakterien ausgewählt ist: *Escherichia coli, Enterococcus faecalis, Providencia stuartii, Klebsiella pneumoniae, Staphylococcus aureus, Staphylococcus epidermidis, Pseudomonas aeruginosa, Klebsiella oxytoca* und *Enterobacter cloacae.*

**14.** Verfahren nach einem der Ansprüche 2 bis 13, **dadurch gekennzeichnet, dass** bei Schritt b) eine Kultivierung mit einem polyvalenten Bakterien-Kulturmedium durchgeführt wird.

**15.** Verfahren nach einem der Ansprüche 4 bis 14, **dadurch gekennzeichnet, dass** bestimmt wird, ob die zu analysierende Probe eines Blutproduktes, vorzugsweise ein Blutplättchen-Konzentrat, mit einer Bakterie, vorzugsweise in einer Konzentration von wenigstens 10 Bakterien/ml, infiziert ist, wenn der anhand der 2 Spektren berechnete Differenzscore variiert, vorzugsweise größer wird, zwischen:

- einem ersten Vergleichsscorewert zwischen einem ersten Spektrum eines Proteinextraktes der untersuchten Probe vor Kultivierung oder nach Kultivierung zum Zeitpunkt t0 und einem zweiten Spektrum eines Proteinextraktes der untersuchten Probe jeweils nach Kultivierung bei t1, wobei t1 größer als t0 ist, wobei vorzugsweise t0 0 bis 6h beträgt und t1 2 bis 24h beträgt, wobei weiterhin vorzugsweise t0 0 bis 2 h beträgt und t1 2 bis 6h beträgt, und

- wenigstens einem zweiten Vergleichsscorewert zwischen einem ersten Spektrum eines Proteinextraktes der untersuchten Probe vor Kultivierung oder nach Kultivierung zum Zeitpunkt t0 und einem zweiten Spektrum eines Proteinextraktes der untersuchten Probe jeweils nach Kultivierung bei t2, wobei t2 größer als t1 ist, wobei vorzugsweise t0 0 bis 2h beträgt, wobei t1 2 bis 6h beträgt und t2 4 bis 16h beträgt.

## Claims

1. A method of detecting contamination by a microbe of a sample of blood product containing proteins and/or macromolecules and/or cells and/or fragments of cells of blood, or for complementing blood, and in which a said microbe can proliferate, said microbe preferably being a bacterium or a yeast, the method comprising the following steps:

   a) performing lysis treatment of at least the said microbe(s) that might be contained in said sample of said blood product for analysis after an incubation time $t$;
   b) using a mass spectrometer, preferably of the Maldi-Tof type, to take a spectrum of said sample of said blood product or of a protein extract from said sample of said blood product as lysed in this way at a time $t$; and
   c) determining whether the sample for analysis is contaminated by comparing the resulting spectrum with at least one spectrum from a Maldi-Tof type mass spectrometer selected from the following spectra:

   c.1) at least one reference spectrum selected from:

   c.1.1 - at least one spectrum of a said standard sample or respectively of a said protein extract of said standard sample, said standard sample or respectively said extract of said standard sample being a sample of the same nature as said sample for analysis, but not contaminated; and
   c.1.2 - spectra of said standard samples or of protein extracts of standard samples of the same nature, each contaminated by a respective known different microbe, said standard samples or said extracts having been subjected to the same lysis treatment; and

   c.2) at least one spectrum of a said original sample or said extract of said original sample that has been subjected to the same lysis treatment before incubation or after being incubated for a time t' different from $t$.

2. A method according to claim 1 for detecting contamination by a microbe, preferably a bacterium, of a sample of said blood product for analysis, by tracking the growth of said microbe, the method comprising the following successive steps:

   a.1) culturing said microbe by adding a culture medium for said microbe to said sample for a time $t$, preferably lying in the range 6 hours (h) to 24 h; and
   a.2) performing cellular lysis of the microbes and other cells of said sample; then
   a.3) extracting proteins from a lysate of said sample after culture from step a.2); and
   b) using a Maldi-Tof type mass spectrometer to take a spectrum of said protein extract obtained in step a.3); and
   c) determining whether said sample is contaminated by comparing the spectrum obtained in step b) with at least one Maldi-Tof type mass spectrometry spectrum selected from the following spectra:

   c.1) at least one reference spectrum selected from:

   - c.1.1) a spectrum of a reference protein extract of a standard sample of the same nature but not contaminated, that has been subjected to the same lysis and extraction treatment of steps a.2) and a.3); and
   - c.1.2) reference spectra of protein extracts of standard samples of the same nature contaminated by known microbes and that have been subjected to the same treatment of at least steps a.1) and a.2), and preferably that have been subjected to the same treatment of at least steps a.1) to a.3); and

   c.2) at least one spectrum of a protein extract obtained by extracting proteins from a lysis and extraction treatment of said original sample in accordance with steps a.2) to a.3) before or after culturing of step a.1) during a time t' shorter than $t$.

3. A method according to claim 1 or claim 2, **characterized in that** said sample of blood product for analysis is constituted by a complex liquid medium containing eucaryotic cells or fragments of eucaryotic cells, preferably blood cells or fragments of blood cells or bone marrow cells.

4. A method according to any one of claims 1 to 3, **characterized in that** said sample of blood product is a human or animal sample containing all or part of the blood selected from: whole blood; a concentrate of red corpuscles; a concentrate of platelets; or fresh plasma.

5. A method according to claim 4, **characterized in that** said sample is a concentrate of platelets, and in step c), step c.1) is performed by comparing the spectrum of the protein extract of the lysate of the concentrate of platelets for analysis with a reference spectrum that is a spectrum of a protein extract of a standard platelet concentrate that is not contaminated, and contamination is detected if the spectrum of the analyzed sample is found to contain at least the specific peaks of greatest heights of the reference spectrum.

6. A method according to any one of claims 1 to 5, **characterized in that**, in step a), said protein extract is obtained by performing the following successive steps:

1) eliminating liquid from said sample for analysis, and recovering a first solid residue; and
2) preferably, adding a detergent solution to said first solid residue; and
3) eliminating liquid once more and recovering a second solid residue; and
4) performing lysis of the cells present in said second solid residue, preferably chemical lysis; and
5) dissolving the proteins contained in said second solid residue, preferably with a solvent solution; and
6) eliminating the non-dissolved substances in order to recover a solution constituted essentially by whole proteins.

7. A method according to claim 6, **characterized in that** in order to process a sample of concentrate of platelets, said protein extract is prepared by performing at least the following successive steps:

1) eliminating liquid from said sample for analysis, by performing first centrifuging and eliminating the centrifuging supernatant, and recovering therefrom a first solid residue, which is the first centrifuging pellet; and
2) adding a detergent solution to said first solid residue, preferably a solution of saponin; and
3) recovering a second solid residue by performing second centrifuging and eliminating the supernatant therefrom, and recovering a second solid residue, which is the pellet of the second centrifuging; and
4) performing chemical lysis of the cells present in said second solid residue, preferably performing chemical treatment with an acid solution of formic acid; and
5) dissolving the proteins present in said second solid residue of cellular lysate, preferably with a solution of formic acid and acetonitrile; and
6) eliminating the non-dissolved substances by performing third centrifuging and eliminating the pellet, and recovering the supernatant of the third centrifuging in order to recover a solution constituted essentially of whole proteins.

8. A method according to any one of claims 4 to 7, **characterized in that** said specific reference spectrum of said extract of non-contaminated blood platelet concentrate comprises at least the peaks of greatest height in Table 1 below, in which the various characteristic peaks are **characterized by** their m/z abscissa axis coordinates (Da) and by their relative height ordinate axis coordinates expressed in S/N (height of the peak / height of background noise), where the m/z molecular mass values may vary by up to $\pm 1$ Da of the mean value mentioned in Table 1, and the relative height values may vary by up to $\pm 20\%$ of the mentioned relative mean height value:

**Table 1**

| Index | m/z | Height (S/N) | Index | m/z | Height (S/N) |
|---|---|---|---|---|---|
| 2 | 2039.33 | 0.82 | 45 | 5941.47 | 12.96 |
| 3 | 2053.09 | 0.96 | 46 | 6123.11 | 4.61 |
| 4 | 2172.47 | 2.81 | 47 | 6195.2 | 4.99 |
| 5 | 2188.26 | 2.08 | 48 | 6253.84 | 12.31 |

(continued)

| Index | m/z | Height (S/N) | Index | m/z | Height (S/N) |
|---|---|---|---|---|---|
| 6 | 2206.14 | 1.49 | 49 | 6329.64 | 34.15 |
| 7 | 2335.49 | 0.81 | 50 | 6399.07 | 4.69 |
| 8 | 2587.08 | 9.77 | 51 | 6437.89 | 19.1 |
| 9 | 2677.66 | 6.53 | 52 | 6636.41 | 10.92 |
| 10 | 2824.37 | 44.11 | 53 | 6667.28 | 30.17 |
| 11 | 3124.2 | 2.88 | 54 | 6725.81 | 41.44 |
| 12 | 3170.04 | 2.43 | 55 | 7193.2 | 13.26 |
| 13 | 3332.51 | 4.02 | 56 | 7307.12 | 1.57 |
| 14 | 3372.52 | 27.37 | 57 | 7355.33 | 2.03 |
| 15 | 3443.5 | 48.02 | 58 | 7437.27 | 19.85 |
| 16 | 3487.23 | 15.79 | 59 | 7567.73 | 42.56 |
| 17 | 3717.3 | 2.85 | 60 | 7642.27 | 28.22 |
| 18 | 3783.47 | 6.75 | 61 | 7768.13 | 745.58 |
| 19 | 3820.02 | 5.97 | 62 | 7839.16 | 152.91 |
| 20 | 3883.82 | 343.89 | 63 | 7924.93 | 114.32 |
| 21 | 3920.17 | 34.15 | 64 | 8022.69 | 21.31 |
| 22 | 3963.03 | 32.32 | 65 | 8144.94 | 177.92 |
| 23 | 4049.38 | 1.6 | 66 | 8211.76 | 12.77 |
| 24 | 4072.05 | 48.19 | 67 | 8584.21 | 37.68 |
| 25 | 4292.54 | 15.29 | 68 | 8652.46 | 28.04 |
| 26 | 4326.29 | 5.36 | 69 | 8719.83 | 16.44 |
| 27 | 4481.88 | 47.61 | 70 | 8963.52 | 106.86 |
| 28 | 4568.92 | 4.21 | 71 | 9039.83 | 6.86 |
| 29 | 4623.37 | 14.23 | 72 | 9136.49 | 7.45 |
| 30 | 4646.43 | 14.15 | 73 | 9249.43 | 26.67 |
| 31 | 4688.05 | 31.78 | 74 | 9291.19 | 35.04 |
| 32 | 4732.37 | 17.33 | 75 | 9375.12 | 58.79 |
| 33 | 4760.03 | 62.14 | 76 | 9464.76 | 35.82 |
| 34 | 4813.43 | 38.33 | 77 | 9519.48 | 89.66 |
| 35 | 4896.95 | 40.58 | 78 | 9621.13 | 71.71 |
| 36 | 4966.02 | 17.88 | 79 | 10102.91 | 69.65 |
| 37 | 5052.1 | 44.86 | 80 | 10189.05 | -0.33 |
| 38 | 5137.16 | 4.66 | 81 | 10262.85 | 2.29 |
| 39 | 5268.85 | 46.78 | 82 | 10617.08 | 29.7 |
| 40 | 5310.04 | 31.23 | 83 | 10701.45 | 1.97 |
| 41 | 5360.04 | 138.98 | 84 | 10843.44 | 40.58 |
| 42 | 5422.73 | 19.14 | 85 | 12392.91 | 2.15 |
| 43 | 5652.14 | 229.61 | 86 | 12503.41 | 9.72 |

(continued)

| Index | m/z | Height (S/N) | Index | m/z | Height (S/N) |
|---|---|---|---|---|---|
| 44 | 5698.21 | 13.9 | 87 | 13441.97 | 3.28 |

9. A method according to any one of claims 1 to 8, **characterized in that** in step c), the two spectra are compared essentially by calculating the difference between the respective total areas of the two spectra, preferably two spectra of protein extracts of the same original sample after two different incubation or culture times.

10. A method according to claim 9, **characterized in that**, in step c), at least the following successive steps are performed:

i.1) taking the respective heights yi of each of the abscissa points xi corresponding to the mass ratio values m/z of each spectrum; and
i.2) for each xi value calculating a resultant height yi3 by taking the difference between the heights yi1 and yi2 respectively of said spectrum for analysis (yi1) and of said reference spectrum (yi2):

$$yi3 = yi2 - yi1$$

and
i.3) calculating the sum of the resulting heights yi3 for all of the xi values in order to obtain a characterization score referred to as the "differential" score:

$$\sum_{i=0}^{i=n} |yi3|.$$

11. A method according to claim 10, **characterized in that**, in step c), at least the following steps are performed:

i.1) normalizing the respective heights yi of each of the abscissa points xi corresponding to the m/z ratio values of each spectrum by dividing the heights yi of each abscissa point xi (m/z) by the height of the peak of greatest height; and
i.2) for each value of xi, calculating a normalized resulting height yi3 by taking the difference between the respective normalized heights of the two spectra yi1 and yi2;

$$yi3 = yi2 - yi1$$

and
i.3) calculating the sum of the resulting normalized heights yi3 for all values xi in order to obtain a characterization score referred to as the "differential" score:

$$\sum_{i=0}^{i=n} |yi3|$$

and preferably normalizing the sum by dividing it by the sum of the heights for all of the values xi of one of the spectra, preferably the spectrum having the smaller sum of heights for all of the values xi, in order to obtain a normalized differential score:

$$\sum_{i=0}^{i=n} |yi3| / \sum_{i=0}^{i=n} yip$$

with p=1 or 2.

12. A method according to claim 11, **characterized in that** in step i.3), the following prior filtering steps i.3a) and i.3b) are performed before the step 3c):

i.3a) for each value of xi, removing the background noise by giving the value yi3=0 to the resulting normalized height if yi3 is less than a threshold $Sy_1$, where $Sy_1$ is less than 10% of the value $yi_{max}$ of the peak of greatest height, preferably $Sy_1$ = 5% of the value of $yi_{max}$ of the peak of greatest height; and

i.3b) filtering by giving the value yi3=0 for the values of xi for which at xi', with xi'-xi less than a threshold Sx, Sx preferably being less than or equal to 10Da, the resulting normalized height yi3' is of sign opposite to yi3 and of absolute value not less than a relative threshold value of $Sy_2$% of the value of yi3, $Sy_2$ preferably being at least 70% of the value of yi3; and

i.3c) calculating the sum of the heights that remain after yi3 filtering for all of the values xi in order to obtain a characterization score referred to as the "differential" score:

$$\sum_{i=0}^{i=n} |yi3|$$

and preferably normalizing the sum by dividing it by the sum of the heights for all of the values xi of one of the spectra, preferably the spectrum having the smaller sum of heights for all the values xi, in order to obtain a normalized differential score:

$$\sum_{i=0}^{i=n} |yi3| / \sum_{i=0}^{i=n} yip$$

with p=1 or 2.

13. A method according to any one of claims 1 to 12, **characterized in that** a search is made to detect the presence of a bacterium selected at least from the following bacteria: *Escherichia coli, Enterococcus faecalis, Providencia stuartii, Klebsiella pneumoniae, Staphylococcus aureus, Staphylococcus epidermidis, Pseudomonas aeruginosa, Klebsiella oxytoca* and *Enterobacter cloacae.*

14. A method according to any one of claims 2 to 13, **characterized in that** in step b), a culture is prepared with a polyvalent bacterial culture medium.

15. A method according to any one of claims 4 to 14, **characterized in that** it is determined whether the sample of said blood product for analysis, preferably a concentrate of platelets, is infected by a bacterium, preferably at a concentration of at least 10 bacteria per milliliter, if said differential score calculated from the two spectra varies, and preferably increases, between:

• a first comparison score value between a first spectrum of a said protein extract of said sample tested prior to culturing or after culturing for the time t0, and a second spectrum of a said protein extract of said sample tested respectively after culturing for t1, where t1 is greater than t0, preferably with t0 lying in the range 0 to 6 h, and t1 lying in the range 2 h to 24 h, more preferably t0 lying in the range 0 to 2 h and t1 lying in the range 2 h to 6 h; and

• at least one second comparison score value between a first spectrum of a said protein extract of said tested sample before culturing or after culturing for the time t0, and a second spectrum of a said protein extract of said tested sample respectively after culturing for t2, where t2 is greater than t1, with t0 preferably lying in the range 0 to 2 h, t1 lying in the range 2 h to 6 h, and t2 lying in the range 4 h to 16 h.

FIG.1

FIG.2

EP 3 024 942 B1

FIG.3A

FIG.3B

FIG.3C

FIG.4A

FIG.4B

FIG.4C

FIG.4

FIG.5

FIG.6

EP 3 024 942 B1

EP 3 024 942 B1

FIG.7

45

FIG.7A

FIG.7B

FIG.8A

FIG.8B

FIG.9A

FIG.9B